(19) Europäisches Patentamt
European Patent Office
Office européen des brevets

(11) Publication number: **0 395 729 B1**

(12) # EUROPEAN PATENT SPECIFICATION

(45) Date of publication of patent specification: **27.09.95**

(51) Int. Cl.⁶: **C07C 29/48**, C07C 31/20, C07C 33/26

(21) Application number: **89901900.4**

(22) Date of filing: **10.01.89**

(86) International application number: **PCT/US89/00086**

(87) International publication number: **WO 89/06225 (13.07.89 89/15)**

Divisional application 95200458.8 filed on 10/01/89.

(54) **LIGAND-ACCELERATED CATALYTIC ASYMMETRIC DIHYDROXYLATION.**

(30) Priority: **11.01.88 US 142692**
**23.02.88 US 159068**
**28.09.88 US 250378**

(43) Date of publication of application: **07.11.90 Bulletin 90/45**

(45) Publication of the grant of the patent: **27.09.95 Bulletin 95/39**

(84) Designated Contracting States: **AT BE CH DE FR GB IT LI LU NL SE**

(56) References cited:

**Chemical Reviews, vol. 80, no. 2, April 1980, Easton (US) M. Schröder: "Osmium tetraoxide Cis hydroxylation of unsaturated substrates" pages 187-213**

(73) Proprietor: **MASSACHUSETTS INSTITUTE OF TECHNOLOGY**
**77 Massachusetts Avenue**
**Cambridge, MA 02139 (US)**

(72) Inventor: **MARKO, Istvan, E.**
**442 Glossop Road**
**Sheffield S10 2 PX (GB)**
Inventor: **SHARPLESS, K., Barry**
**8 Chilton Street**
**Brookline, MA 02146 (US)**

(74) Representative: **Holdcroft, James Gerald, Dr. et al**
**Graham Watt & Co.,**
**Riverhead**
**Sevenoaks, Kent TN13 2BN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

The Journal of the American Chemical Society, vol. 102, no. 12, 4 June 1980 (Columbus, Ohio, US) S.G. Hentges et al.: "Asymetric induction in the reaction of osmium tetroxide with olefins", pages 4263-4265

Tetrahedron Letters, no. 23, June 1976, Pergamon Press, (Oxford, GB) V.CanRheenen et al.: "An improved catalytic OSO4 oxidation of olefins to cis-1, 2-glycols using tertiary amine oxides as the oxidant", pages 1973-1976

## Description

Background

In nature, the organic constituents of animals, microorganisms and plants are made up of chiral molecules, or molecules which exhibit handedness. Enantiomers are stereoisomers or chiral molecules whose configurations (arrangements of constituent atoms) are mirror images of each other; absolute configurations at chiral centers are determined by a set of rules by which a priority is assigned to each substituent and are designated R and S. The physical properties of enantiomers are identical, except for the direction in which they rotate the plane of polarized light: one enantiomer rotates plane-polarized light to the right and the other enantiomer rotates it to the left. However, the magnitude of the rotation caused by each is the same.

The chemical properties of enantiomers are also identical, with the exception of their interactions with optically active reagents. Optically active reagents interact with enantiomers at different rates, resulting in reaction rates which may vary greatly and, in some cases, at such different rates that reaction with one enantiomer or isomer does not occur. This is particularly evident in biological systems, in which stereochemical specificity is the rule because enzymes (biological catalysts) and most of the substrates on which they act are optically active.

A mixture which includes equal quantities of both enantiomers is a racemate (or racemic modification). A racemate is optically inactive, as a result of the fact that the rotation of polarized light caused by a molecule of one isomer is equal to and in the opposite direction from the rotation caused by a molecule of its enantiomer. Racemates, not optically active compounds, are the products of most synthetic procedures. Because of the identity of most physical characteristics of enantiomers, they cannot be separated by such commonly used methods as fractional distillation (because they have identical boiling points), fractional crystallization (because they are equally soluble in a solvent, unless it is optically active) and chromatography (because they are held equally tightly on a given adsorbent, unless it is optically active). As a result, resolution of a racemic mixture into enantiomers is not easily accomplished and can be costly and time consuming.

Recently, there has been growing interest in the synthesis of chiral compounds because of the growing demand for complex organic molecules of high optical purity, such as insect hormones and pheromones, prostaglandins, antitumor compounds, and other drugs. This is a particularly critical consideration, for example, for drugs, because in living systems, it often happens that one enantiomer functions effectively and the other enantiomer has no biological activity and/or interferes with the biological function of the first enantiomer.

In nature, the enzyme catalyst involved in a given chemical reaction ensures that the reaction proceeds asymmetrically, producing only the correct enantiomer (i.e., the enantiomer which is biologically or physiologically functional). This is not the case in laboratory synthesis, however, and, despite the interest in and energy expended in developing methods by which asymmetric production of a desired chiral molecule (e.g., of a selected enantiomer) can be carried out, there has been only limited success.

In addition to resolving the desired molecule from a racemate of the two enantiomers, it is possible, for example, to produce selected asymmetric molecules by the chiral pool or template method, in which the selected asymmetric molecule is "built" from pre-existing, naturally-occurring asymmetric molecules. Asymmetric homogeneous hydrogenation and asymmetric epoxidation have also been used to produce chiral molecules. Asymmetric hydrogenation is seen as the first manmade reaction to mimic naturally-occurring asymmetric reactions. Sharpless, K.B., Chemistry in Britain, January 1986, pp 38-44; Mosher, H.S. and J.D. Morrison, Science, 221:1013-1019 (1983); Maugh, T.H., Science, 221:351-354 (1983); Stinson, S., Chemistry and Engineering News, :24 (6/2/86).

Presently-available methods of asymmetric synthesis are limited in their applicability, however. Efficient catalytic asymmetric synthesis reactions are very rare; they require a directing group and thus are substrate limited. Because such reactions are rare and chirality can be exceptionally important in drugs, pheromones and other biologically functional compositions, a catalytic method of asymmetric dihydroxylation would be very valuable. In addition, many naturally-occurring products are dihydroxylated or can be easily derived from a corresponding vicinal diol derivative.

Summary of the Invention

The present invention relates to a method of osmium-catalyzed asymmetric addition to an olefin, comprising the step of combining the olefin, a chiral ligand (for example a cinchona alkaloid, e.g.

dihydroquinine or dihydroquinidine, or derivatives thereof), an oxidant and an osmium-containing catalyst in which either (a) the olefin (which may be dissolved in an organic solvent) is slowly added to the combination and/or (b) a soluble carboxylate salt, for example a tetraalkyl (e.g. tetraethyl) ammonium carboxylate (e.g. acetate) is added.

The invention also relates to a chiral ligand selected from:

a. dimethylcarbamoyl dihydroquinidine;
b. benzoyl dihydroquinidine;
c. 4-methoxybenzoyl dihydroquinidine;
d. 4-chlorobenzoyl dihydroquinidine;
e. 2-chlorobenzoyl dihydroquinidine;
f. 4-nitrobenzoyl dihydroquinidine;
g. 3-chlorobenzoyl dihydroquinidine;
h: 2-methoxybenzoyl dihydroquinidine;
i. 3-methoxybenzoyl dihydroquinidine;
j. 2-naphtoyl dihydroquinidine;
k. cyclohexanoyl dihydroquinidine;
l. p-phenylbenzoyl dihydroquinidine;
m. dimethylcarbamoyl dihydroquinine;
n. benzoyl dihydroquinine;
o. 4-methoxybenzoyl dihydroquinine;
p. 4-chlorobenzoyl dihydroquinine;
q. 2-chlorobenzoyl dihydroquinine;
r. 4-nitrobenzoyl dihydroquinine;
s. 3-chlorobenzoyl dihydroquinine;
t. 2-methoxybenzoyl dihydroquinine;
u. 3-methoxybenzoyl dihydroquinine;
v. 2-naphtoyl dihydroquinine;
w. cyclohexanoyl dihydroquinine;
x. p-phenylbenzoyl dihydroquinine;
y. methoxydihydroquinidine; and

a dihydroquinidine ester of the formula

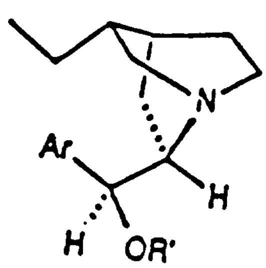

wherein R' is p-chlorobenzoyl and Ar is

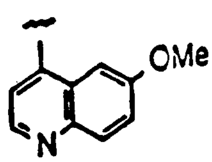

a dihydroquinine ester of the formula

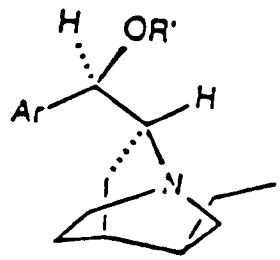

wherein R' is p-chlorobenzoyl and Ar is

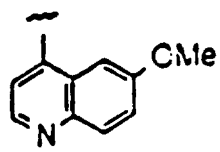

A particular advantage of the catalytic asymmetric method is that only small quantities of osmium catalyst are required.

Brief Description of the Drawings

Figure 1 is a schematic representation of asymmetric dihydroxylation via ligand-accelerated catalysis which is carried out by the method of the present invention.

Figure 2 is a schematic representation of asymmetric catalytic oxyamination of stilbene which is carried out by the method of the present invention.

Figure 3 is a plot of amine concentration vs second-order-rate constant k for the catalytic cis-dihydroxylation of styrene. At point a, no amine has been added. Point a thus represents the rate of the catalytic process in the absence of added amine ligands. Line b represents the rate of the catalytic process in the presence of varying amounts of quinuclidine, a ligand which substantially retards catalysis. Line c represents the rate of the catalytic process in the presence of the dihydroquinidine benzoate derivative 1 represented in Figure 1. K is defined as $K_{obs}/[OsO_4]_o$ where rate = -d[styrene]/dt = $K_{obs}$ [styrene]. Conditions: 25 °C, $[OsO_4]_o$ = 4 x $10^{-4}$ M, $[NMO]_o$ = 0.2M $[styrene]_o$ = 0.1M.

Figure 4 is a schematic representation of a proposed mechanism of catalytic olefin dihydroxylation. This scheme shows two diol-producing cycles believed to be involved in the ligand-accelerated catalysis of the present invention. Formula 1 represents an alkaloid-osmium complex; formula 2 represents a monoglycolate ester; formula 3 represents an osmium(VIII)trioxoglycolate complex; formula 4 represents a bisglycolate osmium ester; and formula 5 represents a dioxobisglycolate. .

Detailed Description of the Invention

Asymmetric epoxidation has been the subject of much research in the past eight years. Earlier work demonstrated that the titanium-tartrate epoxidation catalyst is actually a complex mixture of epoxidation catalysts in dynamic equilibrium with each other and that the main species present (i.e., the 2:2 structure) is the best catalyst (i.e., about six times more active than titanium isopropoxide bearing no tartrate). This work also showed that this rate advantage is essential to the method's success because it ensures that the catalysis is channeled through a chiral ligand-bearing species.

The reaction of osmium tetroxide ($OsO_4$) with olefins is a highly selective and reliable organic transformation. It has long been known that this reaction is accelerated by nucleophilic ligands. Criegee, R. Justus Liebigs Ann. Chem., 522:75 (1936); Criegee, R. et al., Justus Liebigs Ann. Chem., 550:99 (1942); VanRheenen et al., Tetrahedron Lett., 1973 (1976). It has now been shown that a highly effective osmium-catalyzed process can be used to replace previously known methods, such as the stoichiometric asymmetric osmylation method. Hentges, S.G. and K.B. Sharpless, Journal of the American Chemical Society,

102:4263 (1980). The method of the present invention results in asymmetric induction and enhancement of reaction rate by binding of a selected ligand. Through the use of the ligand-accelerated catalytic method of the present invention, asymmetric dihydroxylation, asymmetric diamination or asymmetric oxyamination can be effected.

As a result of this method, two hydroxyl groups are stereospecifically introduced into (imbedded in) a hydrocarbon framework, resulting in cis vicinal dihydroxylation. The new catalytic method of the present invention achieves substantially improved rates and turnover numbers (when compared with previously-available methods), as well as useful levels of asymmetric induction. In addition, because of the improved reaction rates and turnover numbers, less osmium catalyst is needed in the method of the present invention than in previously-known methods. As a result, the expense and the possible toxicity problem associated with previously-known methods are reduced.

The method of the present invention is exemplified below with particular reference to its use in the asymmetric dihydroxylation of E-stilbene ($C_6H_5CH{:}CHC_6H_5$) and trans-3-hexene ($CH_3CH_2CH{:}CHCH_2CH_3$). The method can be generally described as presented below and that description and subsequent exemplification not only demonstrate the dramatic and unexpected results of ligand-accelerated catalysis, but also make evident the simplicity and effectiveness of the method.

The asymmetric dihydroxylation method of the present invention is represented by the scheme illustrated in Figure 1. According to the method of the present invention, asymmetric dihydroxylation of a selected olefin is effected as a result of ligand-accelerated catalysis. That is, according to the method, a selected olefin is combined, under appropriate conditions, with a selected chiral ligand (which in general will be a chiral substituted quinuclidine), an organic solvent, water, an oxidant and osmium tetroxide and, optionally, an acetate compound. In one embodiment, a selected olefin, a chiral ligand, an organic solvent, water and an oxidant are combined; after the olefin and other components are combined, $OsO_4$ is added. The resulting combination is maintained under conditions (e.g., temperature, agitation, etc.) conducive for dihydroxylation of the olefin to occur. Alternatively, the olefin, organic solvent, chiral ligand, water and $OsO_4$ are combined and the oxidant added to the resulting combination. These additions can occur very close in time (i.e., sequentially or simultaneously).

In one aspect of the present invention, components of the reaction mixture are combined, to form an initial reaction combination, and olefin is added slowly to it, generally with frequent or constant agitation, such as stirring. In this aspect, designated the "slow addition" method, organic solvent, chiral ligand, water, $OsO_4$ and the oxidant are combined. The olefin can then be slowly added to the other reactants. It is important that agitation, preferably stirring, be applied during the olefin addition. Surprisingly, for many, if not most olefins, slow addition of the olefin to the initial combination results in much better enantiomeric excess (ee), and a faster rate of reaction than the above-described method (i.e., that in which all the olefin is present at the beginning of the reaction). The beneficial effects (i.e., higher ee's) of slow olefin addition are shown in Table 3 (Column 6). A particular advantage of this slow-addition method is that the scope of the types of olefins to which the asymmetric dihydroxylation method can be applied is greatly broadened. That is, it can be applied to simple hydrocarbon olefins bearing no aromatic substituents, or other functional groups. In this process, the olefin is added slowly (e.g., over time), as necessary to maximize ee. This method is particularly valuable because it results in higher ee's and faster reaction times.

In another aspect of the present method, an additive which accelerates hydrolysis of the osmate ester intermediates is added to the reaction combination. These additives can be soluble, carboxylic acid salts with organic-solubilizing counter-ions (e.g., tetraalkyl ammonium ions). Carboxylate salts which are preferred in the present reaction are soluble in organic media and in organic/aqueous co-solvent systems. For example, tetraethyl ammonium acetate has been shown to enhance the reaction rate and ee of some olefins (Table 3). The additive does not replace the alkaloid in the reaction. Compounds which can be used include benzyltrimethylammoniumacetate, tetramethylammonium acetate and tetraethylammonium acetate. However, other oxyanion compounds (e.g., sulfonates or phosphates) may also be useful in hydrolyzing the osmate ester intermediates. The compound can be added to the reaction combination of organic solvent, chiral ligand, water and $OsO_4$ in a reaction vessel, before olefin addition. It is important to agitate (e.g., by stirring) the reaction combination during olefin addition. The additive can also be added to the reaction combination, described above, wherein all of the olefin is added at the beginning of the reaction. In one embodiment, the amount of additive is generally approximately 2 equivalents; in general from about 1 to about 4 equivalents will be used.

In one embodiment of the present invention, the process can be run in an organic non-polar solvent such as toluene. This embodiment is particularly useful in the slow addition method. Preferably, a carboxylate compound which accelerates hydrolysis of the osmate ester intermediates (e.g., tetraethyl- or tetramethyl ammonium acetate) is added. This embodiment is designated the "phase transfer" method. In

this embodiment, solid olefins, which are not soluble, or have limited solubility, in mixtures of acetone/water or acetonitrile/water, are dissolved in toluene and then added slowly a mixture of organic solvent, chiral ligand, water and $OsO_4$. The carboxylate salt serves the dual function of solubilizing the acetate ion in the organic phase where it can promote hydrolysis of the osmate ester, and carrying water associated with it into the organic phase, which is essential for hydrolysis. Higher ee's are obtained with many substrates using this method.

In a further embodiment of the present invention, a boric acid or a boric acid derivative ($R\text{-}B(OH)_2$, R = alkyl, aryl or OH), such as boric acid itself (i.e., $B(OH)_3$) or phenylboric acid (i.e., $Ph\text{-}B(OH)_2$), can be added to the reaction mixture. In the slow addition method, the boric acid is added to the ligand - organic solvent - $OsO_4$ mixture prior to the addition of the olefin. The amount of boric acid added is an amount sufficient to form the borate ester of the diol produced in the reaction. Without wishing to be bound by theory, it is believed that the boric acid hydrolyzes the osmium ester and captures the diols which are generated in the reaction. Neither water nor a soluble carboxylate such as tetraalkyl ammonium carboxylate, is required to hydrolyze the osmium ester in the present borate reaction. Because the presence of water can make the isolation and recovery of water-soluble diols difficult, the addition of a boric acid makes isolation of these diols easier. Especially, in the case of an aryl or alkyl boric acid, it is easy because, in place of the diol, the product is the cyclic borate ester which can be subsequently hydrolyzed to the diol. Iwasawa et al., Chemistry Letters, pp. 1721-1724 (1988). The addition of a boric acid is particularly useful in the slow addition method.

The amount of water added to the reaction mixture is an important factor in the present method. The optimum amount of water to be added can be determined empirically and, in general, should be that amount which results in maximum ee. Generally, approximately 10 to 16 equivalents of water can be added, preferably 13 to 14 equivalents should be used.

An olefin of interest can undergo asymmetric dihydroxylation according to the present invention. For example, any hydrocarbon containing at least one carbon-carbon double bond as a functional group can be asymmetrically dihydroxylated according to the subject method. The method is applicable to any olefin of interest and is particularly well suited to effecting asymmetric dihydroxylation of prochiral olefins (i.e., olefins which can be converted to products exhibiting chirality or handedness). In the case in which the method of the present invention is used to asymmetrically dihydroxylate a chiral olefin, one enantiomer will be more reactive than the other. As a result, it is possible to separate or kinetically resolve the enantiomorphs. That is, through use of appropriately-selected reactants, it is possible to separate the asymmetrically dihydroxylated product from the unreacted starting material and both the product and the recovered starting material will be enantiomerically enriched.

The chiral ligand used in the asymmetric dihydroxylation method will generally be an alkaloid, or a basic nitrogenous organic compound, which is generally heterocyclic and found widely occurring in nature. Examples of alkaloids which can be used as the chiral ligand in the asymmetric dihydroxylation method include cinchona alkaloids, such as quinine, quinidine, cinchonine, and cinchonidine. Examples of alkaloid derivatives useful in the method of the present invention are shown in Table 1. As described in detail below, the two cinchona alkaloids quinine and quinidine act more like enantiomers than like diastereomers in the scheme represented in Figure 1.

As represented in Figure 1, and as shown by the results in Table 2, dihydroquinidine derivatives (represented as DHQD) and dihydroquinine derivatives (represented as DHQ) have a pseudo-enantiomeric relationship in the present method (DHQD and DHQ are actually diastereomers). That is, they exhibit opposite enantiofacial selection. Such derivatives will generally be esters, although other forms can be used. When dihydroquinidine is used as the ligand, delivery of the two hydroxyl groups takes place from the top or upper face (as represented in Figure 1) of the olefin which is being dihydroxylated. That is, in this case direct attack of the re- or re,re- face occurs. In contrast, when the dihydroquinine derivative is the ligand used, the two hydroxyl groups are delivered from the bottom or lower (si- or si,si-face) face of the olefin, again as represented in Figure 1. This is best illustrated by reference to entries 1, 2 and 5 of Table 2. As shown, when DHQD (dihydroquinidine esters) is used, the resulting diol has an R or R,R configuration and when ligand 2 (dihydroquinine esters) is used, the resulting diol has an S or S,S configuration.

## Table 1 Alkaloid Derivatives

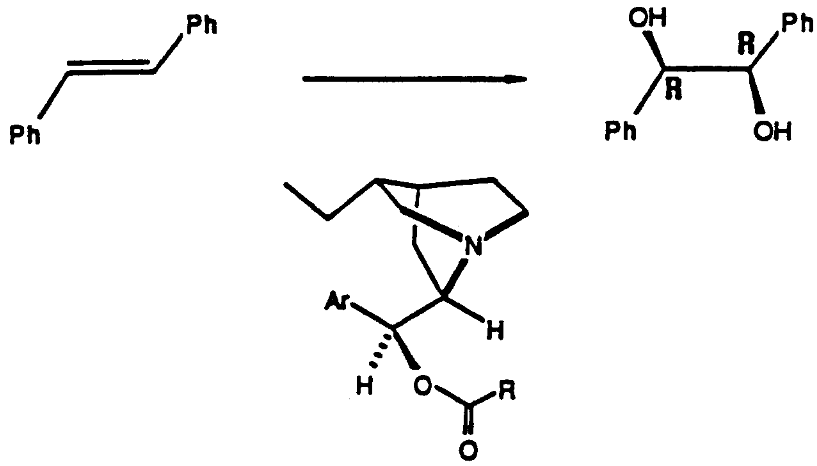

| R | Dihydroquinidine Derivative | Yield (%) | %ee |
|---|---|---|---|
| 3-$ClC_6H_4$ | 3-chlorobenzoyl | 89 | 96.5 |
| 2-$MeOC_6H_4$ | 2-methoxybenzoyl | 89 | 96 |
| 3-$MeOC_6H_4$ | 3-methoxybenzoyl | 87 | 96.7 |
| 2-$C_{10}H_7$ | 2-naphthoyl | 95.4 | 98.6 |
| $C_6H_{11}$ | cyclohexanoyl | 90 | 91 |
| 4-$PhC_6H_4$ | 4-phenylbenzoyl | 89 | 96 |
| 2,6-$(MeO)_2C_6H_3$ | 2,6-dimethoxy-benzoyl | 88 | 92 |
| 4-$MeOC_6H_4$ | 4-methoxybenzoyl | 91 | 97.6 |
| 4-$ClC_6H_4$ | 4-chlorobenzoyl | 93 | 99 |
| 2-$ClC_6H_4$ | 2-chlorobenzoyl | 87 | 94.4 |
| 4-$NO_2C_6H_4$ | 4-nitrobenzoyl | 71 | 93 |
| $C_6H_5$ | benzoyl | 92 | 98 |

Table 1 (cont'd)

| | | | |
|---|---|---|---|
| $Me_2N$ | dimethyl-carbamoyl | 96 | 95 |
| Me | acetyl | 72 | 94 |
| $MeOCH_2$ | α-methoxyacetyl | 66 | 93 |
| $AcOCH_2$ | α-acetoxyacetyl | 96 | 82.5 |
| $Me_3C$ | trimethylacetyl | 89 | 86.5 |

The example below is a phosphoryl derivative and therefore differs from the carboxylic acid ester derivatives shown above: the phosphorus atom is directly bound to the oxygen atom of the alkaloid.

| | | | |
|---|---|---|---|
| $Ph_2P(O)$ | diphenylphosphinic ester | 69 | 97.5 |

Table 2

| Olefins | ligand; ee[a]; confgn. of diol |
|---|---|
| 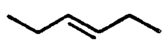 | DHQD; 20%, (70%, 10h); RR<br>DHQ; (60%, 16h); SS |
| 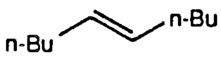 | DHQD; (70%, 120h) |
| 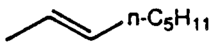 | DHQD; (69%, 30h); RR<br>DHQ; (63%, 30h); SS |
| 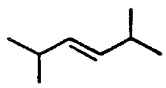 | DHQD; 12%, (46%, 24h),<br>(76%, 24h + 1 eq OAc) |
| 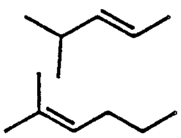 | DHQD; 37.5% |
| | DHQD; (46%, 24h, rt) |
| 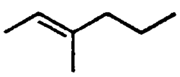 | DHQD; (40%, 24h, rt) |
| 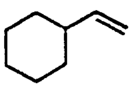 | DHQD; 46%, (50%, 20h); R |
| 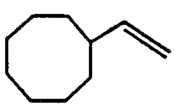 | DHQD; 50% |
| 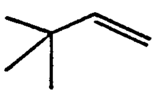 | DHQD; 40% |
| 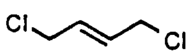 | DHQD; 35%, (40%, 12h) |

[a] Enantiomeric excesses in parentheses were obtained with slow addition of olefin over a period of time indicated and with stirring at O°C except otherwise stated. Tetraethylammonium acetate tetrahydrate were added in some cases as indicated.

Table 2 (cont'd)

| Olefins | ligand; ee[a]; confgn. of diol |
|---|---|
| 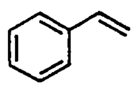 | DHQD; 56%, (61%, 5h); R<br>DHQ; 54%; S |
| 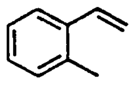 | DHQD; 53% |
| 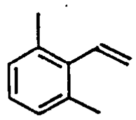 | DHQD; 65% |
| 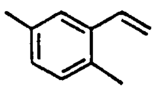 | DHQD; 63% |
| 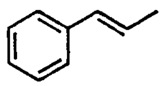 | DHQD; 65%, (86%, 5h); RR<br>DHQ; 55%, (80%, 5h); SS |
| 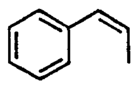 | DHQD; 0-10% |
| 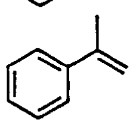 | DHQD; 33%; R |
| 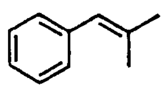 | DHQD; 34%, (53%, 24h) |
| 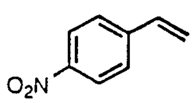 | DHQD; 51% |
| 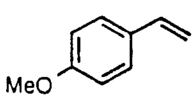 | DHQD; 67% |

[a] Enantiomeric excesses in parentheses were obtained with slow addition of olefin over a period of time indicated and with stirring at O°C except otherwise stated. Tetraethylammonium acetate tetrahydrate were added in some cases as indicated.

Table 2 (cont'd)

| Olefins | ligand; ee[a]; confgn. of diol |
|---|---|
| 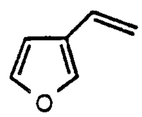 | DHQD; 40% |
| 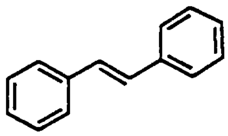 | DHQD; 80%; 92% in the presence of 2 eq. OAc; RR<br>DHQ; 79%; SS |
| 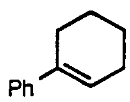 | DHQD; 10%, (78%, 26h),<br>(81%, 16h + 1eq OAc)<br>DHQ; (73%, 26h) |
| 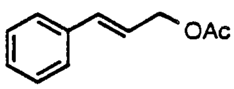 | DHQD; 76%; RR |
| 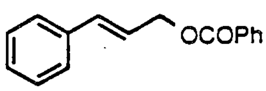 | DHQD; 80% |
| 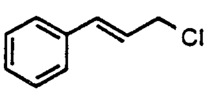 | DHQD; 60%, (78%, 10h) |
| 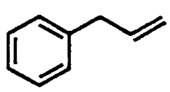 | DHQD; 20% |
| 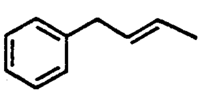 | DHQD; (44%, 10h) |
| 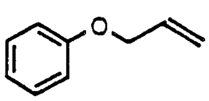 | DHQD; 34% |
| 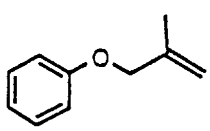 | DHQD; 27% |

[a] Enantiomeric excesses in parentheses were obtained with slow addition of olefin over a period of time indicated and with stirring at 0°C except otherwise stated. Tetraethylammonium acetate tetrahydrate were added in some cases as indicated.

Table 2 (cont'd)

| Olefins | ligand; ee[a]; |
|---|---|
| 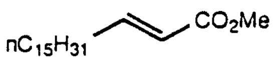 | DHQD; 38% |
| 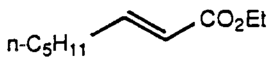 | DHQD; 47.4%, (67%, 31h) |
| 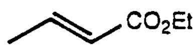 | DHQD; 53% |
| 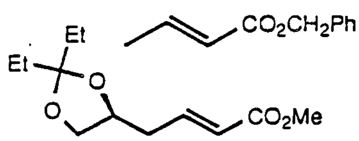 | DHQD; 45% |
| | DHQD; (52% de, 31h) |
| 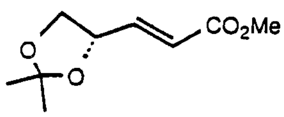 | DHQD; (70% de, 42h) |
| 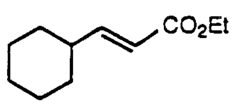 | DHQD; 74.3% |
| 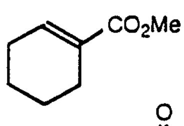 | DHQD; (36%, 24h + OAc, rt) |
| 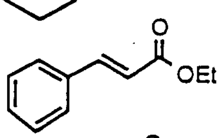 | DHQD; 92% |
| 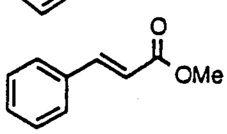 | DHQD; 91% |
| 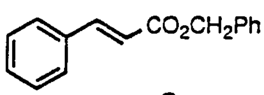 | DHQD; 80-85% |
| 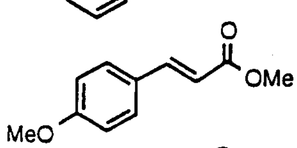 | DHQD; <60%, (80%, slow addition) |
| 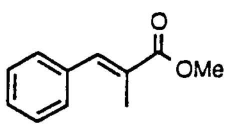 | DHQD; (38%, toluene-water, 24h + OAc, rt) |

[a] Enantiomeric excesses in parentheses were obtained with slow addition of olefin over a period of time indicated and with stirring at O°C except otherwise stated. Tetraethylammonium acetate tetrahydrate were added in some cases as indicated.

Table 2 (cont'd)

| Olefins | ligand; ee[a]; |
|---|---|
| 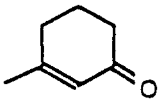 | DHQD; (10%, 24h, rt) |
| 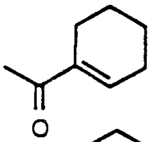 | DHQD; (36%, 24h + OAc, rt) |
| 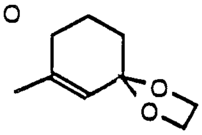 | DHQD; (37%, 12h + OAc) |
| 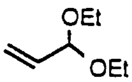 | DHQD; 27%, (31%, 13h) |
| 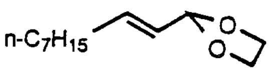 | DHQD; (56%, 20h)<br>(66%, 20h + OAc) |
| 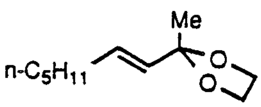 | DHQD; (46%, 18h)<br>(50%, 18h + OAc) |
| 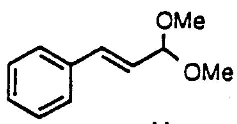 | DHQD; (75%, 18h)<br>(83%, 18h + OAc) |
| 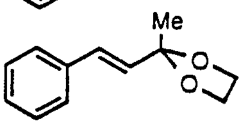 | DHQD; (60%, 10h)<br>(89%, 10h + OAc) |
| 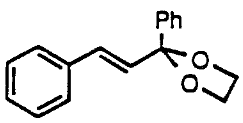 | DHQD; (85%, 20h)<br>(87%, 20h + OAc) |
| 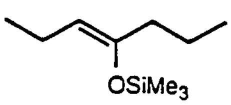 | DHQD; (27%, 23h + OAc) |
| 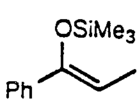 | DHQD; (72%, 23h)<br>(78%, 23h + OAc) |

[a] Enantiomeric excesses in parentheses were obtained with slow addition of olefin over a period of time indicated and with stirring at $0^{\circ}C$ except otherwise stated. Tetraethylammonium acetate tetrahydrate were added in some cases as indicated.

Table 3. Enantiomeric excesses obtained in the asymmetric dihydroxylation of olefins under different conditions

| entry | olefin | stoichiometric[a] | catalytic[b] (original) | catalytic[c] (acetate) | catalytic[d] (slow addition) |
|---|---|---|---|---|---|
| 1 | 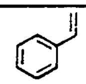 | 61 | 56 | 61 | 60 (5 h) |
| 2 | 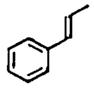 | 87 | 65 | 73 | 86 (5h) |
| 3 | 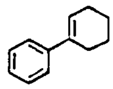 | 79 | 8[e] | 52 | 78 (26 h)[f] |
| 4 | 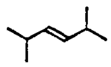 | 80 | 12[g] | 61 | 46 (24 h)[h] |
| | | | | | 76 (24 h + OAc) |
| 5 | 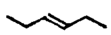 | 69 | 20 | 64 | 70 (10 h) |

[a]All stoichiometric reactions were carried out in acetone-water, 10:1 v/v, at 0 °C and at a concentration of 0.15 M in each reagent. [b]All reactions were carried out at 0 °C according to the original procedure reported in ref. 1(a). [c]All reactions were carried out exactly as described in ref. 1(a) (i.e. *without* slow addition) except that 2 eq of $Et_4NOAc \cdot 4H_2O$ were present. [d]All reactions were carried out at 0 °C as described in note 2 for *trans*-3-hexene with an alkaloid concentration of 0.25 M. The period for slow addition of the olefin is indicated in parentheses. The ee's shown in the Table were obtained with dihydroquinidine *p*-chlorobenzoate as the ligand. Under the same conditions, the pseudoenantiomer, dihydroquinine *p*-chlorobenzoate, provides products with ee's 5-10% lower. In all cases the isolated yield was 85-95%. [e]This reaction took 7 days to complete. [f]With an addition period of 16 h, ee's of 63 and 65% were obtained at 0 °C and 20 °C, respectively; with the combination of slow addition over a period of 16 h and the presence of 1 eq of $Et_4NOAc \cdot 4H_2O$ at 0°C, an ee of 81% was realized. [g]This reaction took 5 days to complete. [h]When the reaction was carried out at 20 °C and the olefin was added over a period of 24 h, an ee of 59% was obtained.

Reference 1(a): Jacobsen et al., JACS, 110:1968 (1988)

Because of this face selection rule or phenomenon, it is possible, through use of the present method and the appropriate chiral ligand, to pre-determine the absolute configuration of the dihydroxylation product.

As is also evident in Table 2, asymmetric dihydroxylation of a wide variety of olefins has been successfully carried out by means of the present invention. Each of the embodiments described results in asymmetric dihydroxylation, and the "slow addition" method is particularly useful for this purpose. In each of the cases represented in the Table in which absolute configuration was established, the face selection "rule" (as interpreted with reference to the orientation represented in Figure 1) applied: use of DHQD resulted in attack or dihydroxylation occurring from the top or upper face and use of DHQ resulted in attack or dihydroxylation occurring from the bottom or lower face of the olefin. This resulted, respectively, in formation of products having an R or R,R configuration and products having an S or S,S configuration.

In general, the concentration of the chiral ligand used will range from 0.01 M to 2.0 M. In one embodiment, exemplified below, the solution is 0.261M in alkaloid 1 (the dihydroquinidine derivative). In one embodiment of the method, carried out at room temperature, the concentrations of both alkaloids represented in Figure 1 are at 0.25M. In this way, the enantiomeric excess resulting under the conditions used is maximized. The amount of chiral ligand necessary for the method of the present invention can be varied as the temperature at which the reaction occurs varies. For example, it is possible to reduce the amount of

alkaloid (or other chiral ligand) used as the temperature at which the reaction is carried out is changed. For example, if it is carried out, using the dihydroquinidine derivative, at 0 ° C, the alkaloid concentration can be 0.15M. In another embodiment, carried out at 0 ° C, the alkaloid concentration was 0.0625M.

Many oxidants (i.e., essentially any source of oxygen) can be used in the present method. For example, amine oxides (e.g., trimethyl amine oxides), tert-butyl hydroperoxide, hydrogen peroxide, and oxygen plus metal catalysts (e.g., copper ($Cu^{+}$-$Cu^{++}/O_2$), platinum ($Pt/O_2$), palladium ($Pd/O_2$) can be used. In one embodiment of the invention, N-methylmorpholine N-oxide (NMO) is used as the oxidant. NMO is available commercially (e.g., Aldrich Chemicals, 97% NMO anhydrous, or as a 60% solution in water).

Osmium will generally be provided in the method of the present invention in the form of osmium tetroxide ($OsO_4$), although other sources (e.g., osmium trichloride anhydrous, osmium trichloride hydrate) can be used. $OsO_4$ can be added as a solid or in solution.

The osmium catalyst used in the method of the present invention can be recycled, for re-use in subsequent reactions. This makes it possible not only to reduce the expense of the procedure, but also to recover the toxic osmium catalyst For example, the osmium catalyst can be recycled as follows: Using reduction catalysts (e.g., Pd-C), the osmium VIII species is reduced and adsorbed onto the reduction catalyst. The resulting solid is filtered and resuspended. NMO (or an oxidant), the alkaloid and the substrate (olefin) are added, with the result that the osmium which is bound to the Pd/C solid is reoxidized to $OsO_4$ and re-enters solution and plays its usual catalytic role in formation of the desired diol. This procedure (represented below) can be carried out through several cycles, thus re-using the osmium species. The palladium or carbon can be immobilized, for example, in a fixed bed or in a cartridge.

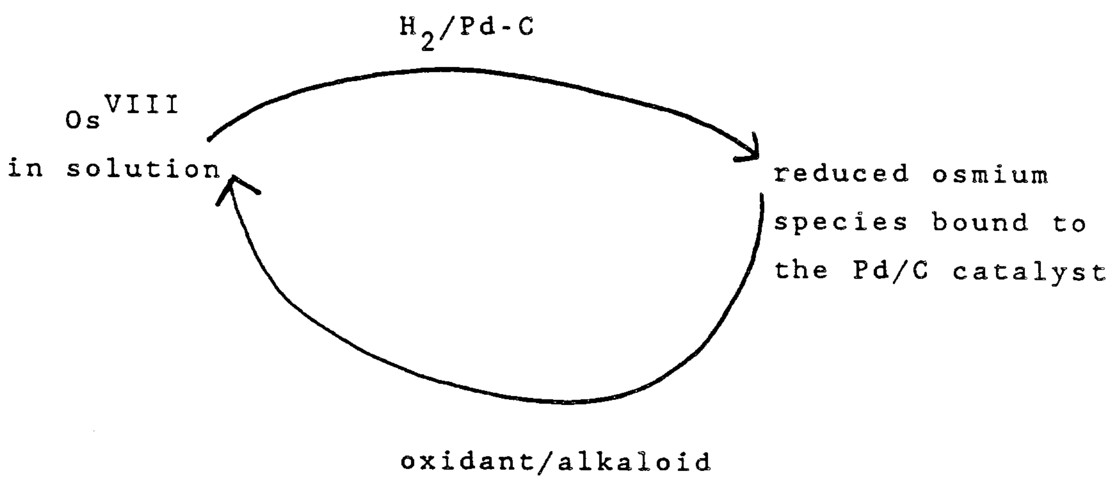


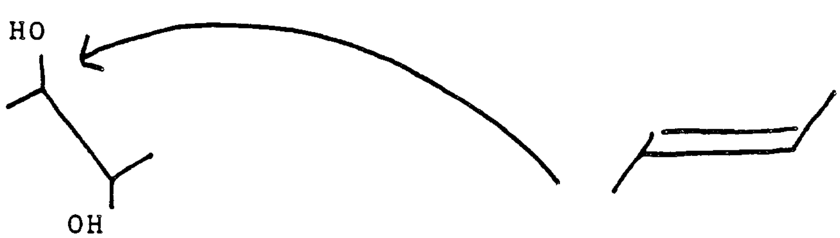

In another preferred embodiment, a chiral ligand (e.g., dihydroquinidine 4-chlorobenzoate), NMO, acetone, water and $OsO_4$ (as a 5M toluene solution) are combined. The components can be added sequentially or simultaneously and the order in which they are combined can vary. In this embodiment, after the components are combined, the resulting combination is cooled (e.g., to approximately 0 ° C); cooling can be carried out using an ice-water bath. It is particularly preferred that the combination is agitated (e.g., stirred). To this well-stirred mixture, an olefin (e.g., trans-3-hexene) is added slowly (e.g., by injection). The optimum rate of addition (i.e., giving maximum ee), will vary depending on the nature of the olefinic substrate. In the case of trans-3-hexene, the olefin was added over a period of about 16-20 hours. After olefin addition, the mixture can be stirred for an additional period of time at the low temperature (1 hour in

the case of trans-3-hexene). The slow-addition method is preferred as it results in better ee and faster reaction times.

In another embodiment, a compound which accelerates hydrolysis of the osmate ester intermediates (e.g., a soluble carboxylate salt, such as tetraethylammonium acetate) is added to the reaction mixture. The compound (approximately 1-4 equiv.) can be added to the mixture of chiral ligand, water, solvent, oxidant and osmium catalyst and olefin, or prior to the addition of olefin, if the olefin slow-addition method is used.

The diol-producing mechanistic scheme which is thought to operate when the slow-addition of olefin method is used is represented in Figure 4. According to the proposed mechanism, at least two diol-producing cycles exist. As shown in Figure 4, only the first cycle appears to result in high ee. The key intermediate is the osmium (VIII) trioxoglycolate complex, shown as formula 3 in Figure 4, which has the following general formula:

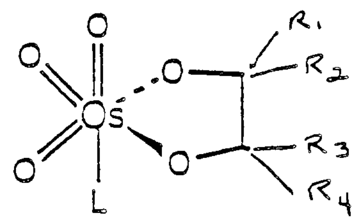

wherein L is a chiral ligand and wherein $R_1$, $R_2$, $R_3$ and $R_4$ are organic functional groups corresponding to the olefin. For example, $R_1$, $R_2$, $R_3$ and $R_4$ could be alkyl, aryl, alkoxy, aryloxy or other organic functional groups compatible with the reaction process. Examples of olefins which can be used, and their functional groups, are shown on Table 2 hereinabove.

This complex occupies the pivotal position at the junction between the two cycles, and determines how diol production is divided between the cycles.

Evidence in favor of the intermediacy of the osmium (VIII) trioxoglycolate complex (formula 3, Figure 4) is provided by the finding that the events in Figure 4 can be replicated by performing the process in a stepwise manner under stoichiometric conditions. These experiments were performed under anhydrous conditions in toluene. In the process shown in Figure 4, one equivalent of the alkaloid osmium complex (shown as formula 1, Figure 4) is allowed to react with an olefin to give the emerald green monoglycolate ester (formula 2, Figure 4). A different olefin is then added, followed by an equivalent of an anhydrous amine N-oxide, and rapid formation of the bisglycolate ester (formula 4, Figure 4) is observed. Upon reductive hydrolysis of the bisglycolate ester, precisely one equivalent of each diol is liberated. These experiments indicate that a second cycle, presumably via the osmium trioxoglycolate complex, is as efficient as the first in producing diols from olefins. One can also use the same olefin in both steps to run this tandem addition sequence. When this was done using 1-phenylcyclohexene as the olefin, the ee for the first step was 81% and the ee for the second step was 7% in the opposite direction (i.e., in favor of the minor enantiomer in the first step). Thus, for this substrate any intrusion of the second cycle is particularly damaging, and under the original catalytic conditions 1-phenylcyclohexene only gave 8% ee (entry 3, Table 3).

Reduced ee is just part of the counterproductivity of turning on the second cycle; reduced turnover is the other liability. The bisosmate esters (formula 4, Figure 4) are usually slow to reoxidize and hydrolyze, and therefore tend to tie up the catalyst. For example, 1-phenylcyclohexene took 7 days to reach completion under the original conditions (the 8% ee cited above). With slow addition of the olefin, the oxidation was complete in one day and gave the diol in 95% yield and 78% ee (entry 3, Table 3).

The most important prediction arising from the mechanistic scheme shown in Figure 4 is the minimization of the second cycle if the olefin is added slowly. Slow addition of the olefin presumably gives the osmium (VIII) trioxoglycolate intermediate sufficient time to hydrolyze so that the osmium catalyst does not get trapped into the second cycle by reacting with olefin. To reiterate, the second cycle not only ruins the ee but also impedes turnover, since some of the complexes involved are slow to reoxidize and/or hydrolyze. The optimum feed rate depends on the olefin; it can be determined empirically, as described herein.

The maximum ee obtainable in the catalytic process is determined by the addition of the alkaloid osmium complex (formula 1, Figure 4) to the olefin (i.e., the first column in Table 3). Thus, stoichiometric additions can be used to enable one to determine the ee-ceiling which can be reached or approached in the

catalytic process if the hydrolysis of 3 (Figure 4) can be made to dominate the alternative reaction with a second molecule of olefin to give 4 (Figure 4). In the case of terminal olefins, styrene (Table 3), the trioxoglycolate esters hydrolyze rapidly, since slow addition, or the effect of the osmate ester hydrolytic additive give only a slight increase in the ee. However, most olefins benefit greatly from any modification which speeds hydrolysis of the osmate ester intermediate (3, Figure 4) (entries 2-5, Table 3), and in extreme cases neither the effect of the osmate ester-hydrolytic additive nor slow addition is sufficient alone. Diisopropyl ethylene (entry 4, Table 3) approaches its ceiling-ee only when both effects are used in concert, with slow addition carried out in the presence of acetate. The other entries in the Table reach their optimum ee's through slow addition alone, but even in these cases the addition times can be substantially shortened if a compound, such as a tetraalkyl ammonium acetate, is present.

In many cases, temperature also effects the ee. When the ee is reduced by the second cycle, raising the temperature can often increase it. For example, diisopropyl ethylene gave 46% ee at 0 °C and 59% ee at 25 °C (24h slow addition time in both cases). The rate of hydrolysis of the osmium trioxoglycolate intermediate is apparently more temperature dependent than the rate of its reaction with olefin. This temperature effect is easily rationalized by the expected need to dissociate the chiral ligand from the osmium complex (3) in order to ligate water and initiate hydrolysis, but the ligand need not dissociate for addition of olefin to occur (in fact this second cycle olefin addition step is also likely to be ligand-accelerated).

The following is a description of how optimum conditions for a particular olefin can be determined. To optimize the osmium-catalyzed asymmetric dihydroxylation: 1) If from the known examples there is doubt about what the ceiling-ee is likely to be, it can be determined by performing the stoichiometric osmylation in acetone/water at 0 °C using one equivalent of the $OsO_4$ - alkaloid complex; 2) Slow addition at 0 °C: the last column in Table 1 can be used as a guide for choosing the addition time, bearing in mind that at a given temperature each olefin has its own "fastest" addition rate, beyond which the ee suffers as the second cycle turns on. When the olefin addition rate is slow enough, the reaction mixture remains yellow-orange (color of 1, Figure 4); when the rate is too fast, the solution takes on a blackish tint, indicating that the dark-brown-to-black bisglycolate complex (4, Figure 4) is being generated; 3) If the ceiling ee is not reached after steps 1 and 2, slow addition plus tetraalkyl ammonium acetate (or other compound which assists hydrolysis of the osmate ester intermediate) at 0 °C can be used; 4) slow addition plus a soluble carboxylate salt, such as tetraalkyl ammonium acetate at room temperature can also be used. For all these variations, it is preferable that the mixture is agitated (e.g., stirred) for the entire reaction period.

The method of the present invention can be carried out over a wide temperature range and the limits of that range will be determined, for example, by the limit of the organic solvent used. The method can be carried out, for example, in a temperature range from about 40 °C to about -30 °C. Concentrations of individual reactants (e.g., chiral ligand, oxidant, etc.) can be varied as the temperature at which the method of the present invention is carried out. The saturation point (e.g., the concentration of chiral ligand at which results are maximized) is temperature-dependant. As explained previously, for example, it is possible to reduce the amount of alkaloid used when the method is carried out at lower temperatures.

The organic solvent used in the present method can be, for example, acetone, acetonitrile, THF, DME, ethanol, methanol, pinacolone, tert butanol or a mixture of two or more organic solvents.

Using the methods described in the Exemplification, HPLC analysis demonstrated that the enantiomeric excess of the resulting diol was 78%.

In another embodiment of the present invention, styrene was combined with a chiral ligand (DHQD), acetone, water and NMO and $OsO_4$. The plot of amine concentration vs second-order-rate-constant K for the catalytic cis-dihydroxylation of styrene is represented in Figure 3. The kinetic data of Figure 3 clearly shows the dramatic effect of ligand-accelerated catalysis achieved by use of the method of the present invention. Point a in Figure 3 represents the rate of the catalytic process in the absence of amine ligands (t1/2 = 108 minutes). Line b shows the rates of the process in the presence of varying amounts of quinuclidine, a ligand which substantially retards catalysis (at greater than 0.1M quinuclidine, t1/2 is greater than 30 hours). Because of the observed retarding effect of quinuclidine (ligand-decelerated catalysis) the result represented by line C was unexpected. That is, when the process occurs in the presence of dihydroquinidine benzoate derivative 1 (see Figure 1), the alkaloid moiety strongly accelerates the catalytic process at all concentrations (with ligand 1 = 0.4M, t1/2 = 4.5 minutes), despite the presence of the quinuclidine moiety in its structure.

The rate of the stoichiometric reaction of styrene with osmium tetroxide and that of the corresponding catalytic process were compared. The comparison indicates that both have identical rate constants [$K_{stoic}$ = -(5.1 ± 0.1)x$10^2$$M^{-1}$ $min^{-1}$ and $K_{cat}$ = (4.9 ± 0.4)x$10^2$$M^{-1}$ $min^{-1}$], and that they undergo the same rate acceleration upon addition of ligand 1. Hydrolysis and reoxidation of the reduced osmium species, steps

which accomplish catalyst turnover, are not kinetically significant in the catalytic process with styrene. It may be concluded that the limiting step is the same in both processes and consists of the initial addition reaction forming the osmate ester (2, Figure 4). A detailed mechanistic study reveals that the observed rate acceleration by added ligand 1 is due to formation of an osmium tetroxide-alkaloid complex which, in the case of styrene, is 23 times more reactive than free osmium tetroxide. The rate reaches a maximal and constant value beyond an (approximate) 0.25 M concentration of ligand 1. The onset of this rate saturation corresponds to a pre-equilibrium between DHQD and osmium tetroxide with a rather weak binding constant ($K_{eq}$ = 18 ± 2 $M^{-1}$). Increasing the concentration of DHQD above 0.25 M does not result in corresponding increases in the enantiomeric excess of the product diol. In fact, due to the ligand-acceleration effect, the ee of the process approaches its maximum value much faster than the maximum rate is reached, which means that optimum ee can be achieved at rather low alkaloid concentrations.

At least in the case of styrene, the rate acceleration in the presence of the alkaloid is accounted for by facilitation of the initial osmylation step. The strikingly opposite effects of quinuclidine and DHQD on the catalysis can be related to the fact that although quinuclidine also accelerates the addition of osmium tetroxide to olefins, it binds too strongly to the resulting osmium(VI) ester intermediate and inhibits catalyst turnover by retarding the hydrolysis/reoxidation steps of the cycle. In contrast the alkaloid appears to achieve a balancing act which renders it near perfect for its role as an accelerator of the dihydroxylation catalysis. It binds strongly enough to accelerate addition to olefins, but not so tightly that it interferes (as does quinuclidine) with subsequent stages of the catalytic cycle. Chelating tertiary amines [e.g., 2,2'-bipyridine and (-)-(R,R)-N,N,N',N'-tetramethyl-1,2-cyclohexanediamine) at 0.2M completely inhibit the catalysis. Pyridine at 0.2 M has the same effect.

As represented in Table 2, the method of the present invention has been applied to a variety of olefins. In each case, the face selection rule described above has been shown to apply (with reference to the orientation of the olefin as represented in Figure 1). That is, in the case of the asymmetric dihydroxylation reaction in which the dihydroquinidine derivative is the chiral ligand, attack occurs on the re- or re,re- face) and in the case in which the dihydroquinine derivative is the chiral ligand, attack occurs on the si- or si,si-face. Thus, as demonstrated by the data presented in the Table 2, the method of the present invention is effective in bringing about catalytic asymmetric dihydroxylation; in all cases, the yield of the diol was 80-95%, and with the slow-addition modification, most olefins give ee's in the range of 40-90%.

The present method can be used to synthesize chiral intermediates which are important building blocks for biologically active chiral molecules, such as drugs. In one embodiment, the present method was used to produce an optically pure intermediate used in synthesizing the drug diltiazum (also known as cardizem). The reaction is shown in the following scheme:

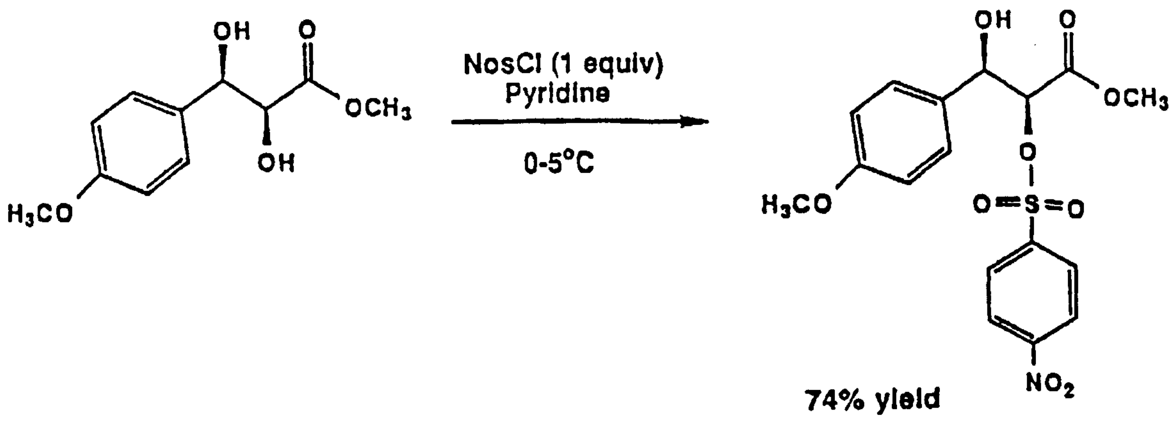


The method of the present invention is also useful to effect asymmetric vicinal oxyamination of an olefin, and may be useful for asymmetric vicinal diamination. In the case of substitution of two nitrogen or of a nitrogen and oxygen, an amino derivative is used as an amino transfer agent and as an oxidant. For example, the olefin to be modified, an organic solvent, water, a chiral ligand, an amino derivative and an osmium-containing compound are combined and the combination maintained under conditions appropriate for the reaction to occur. The amino derivative can be, for example, an N-chlorocarbamate or chloroamine T. Asymmetric catalytic oxyamination of recrystallized trans stilbene, according to the method of the

present invention, is represented in Figure 2.

EXAMPLE I Asymmetric Dihydroxylation of Stilbene

The following were placed sequentially in a 2L bottle (or flask): 180.2g (1.0 M) of recrystallised trans stilbene (Aldrich 96%), 62.4g (0.134 moles; 0.134 eq) of the p-chlorobenzoate of hydroquinidine (1), 450 mL of acetone, 86 mL of water (the solution is 0.261 M in alkaloid 1) and 187.2 g (1.6 mol, 1.6 eq.) of solid N-Methylmorpholine N-Oxide (NMO, Aldrich 97%). The bottle was capped, shaken for 30 seconds, cooled to 0-4°C using an ice-water bath. $OsO_4$ (4.25 mL of a solution prepared using 0.120g $OsO_4$/mL toluene; 0.002 Mol%; 0.002 eq.) was injected. The bottle was shaken and placed in a refrigerator at ca. 4°C with occasional shaking. A dark purple color developed and was slowly replaced by a deep orange one; the heterogeneous reaction mixture gradually became homogeneous and at the end of the reaction, a clear orange solution was obtained. The reaction can be conveniently monitored by TLC (silica gel; $CH_2Cl_2$; disappearance of the starting material at a defined Rf). After 17 hours, 100g of solid sodium metabisulfite ($Na_2S_2O_5$) were added, the reaction mixture was shaken (1 minute) and left at 20°C during 15 minutes. The reaction mixture was then diluted by an equal volume of $CH_2Cl_2$ and anhydrous $Na_2SO_4$ added (100 g). After another 15 minutes, the solids were removed by filtration through a pad of celite, washed three times with 250 mL portions of $CH_2Cl_2$ and the solvent was evaporated under vacuum (rotatory evaporator, bath temperature = 30-35°C).

The crude oil was dissolved in ethyl acetate (750 mL), extracted three times with 500 ml. portions of 2.0 M HCl, once with 2.0 M NaOH, dried over $Na_2SO_4$ and concentrated in vacuo to leave 190 g (89%) of the crude diol as a pale yellow solid. The enantiomeric excess of the crude R,R-diol was determined to be 78% by HPLC analysis of the derived bis-acetate (Pirkle 1A column using 5% isopropanol/hexane mixture as eluant. Retention times are: t1 = 18.9 minutes; t2 = 19.7 minutes. Recrystallization from about 1000 ml. $CH_2Cl_2$ gave 150 g (70%) of pure diol (ee = 90%). A second recrystallization gave 115g of diol (55% yield) of 99% ee. Ee (enantiomeric excess) is calculated from the relationship (for the R enantiomer, for example): percent e.e. = [(R)-(S)/[(R) + (S)]x100.

The aqueous layer was cooled to 0°C and treated with 2.0M NaOH (about 500 mL) until pH = 7. Methylene chloride was added (500 mL) and the pH adjusted to 10-11 using more 2.0M NaOH (about 500 mL). The aqueous layer was separated, extracted twice with methylene chloride (2x300 mL) and the combined organic layers were dried over $Na_2SO_4$. The solvent was removed in vacuo to provide the alkaloid as a yellow foam. The crude alkaloid was dissolved in ether (1000 mL), cooled to 0°C (ice-bath) and treated with dry HCl until acidic pH (about 1-2). The faint yellow precipitate of p-chlorobenzoylhydroquinidine hydrochloride was collected by filtration and dried under high vacuum (0.01mm Hg).

The free base was liberated by suspending the salt in ethyl acetate (500 mL), cooling to 0°C and adding 28% $NH_4OH$ until pH = 11 was reached. After separation, the aqueous layer was extracted twice with ethyl acetate, the combined organic layers were dried over $Na_2SO_4$ and the solvent removed in vacuo to give the free base as a white foam.

EXAMPLE 2 Asymmetric Dihydroxylation of Stilbene

Asymmetric dihydroxylation of stilbene was carried out as described in Example 1, except that 1.2 equivalents of NMO were used.

EXAMPLE 3 Asymmetric Dihydroxylation of Stilbene

Asymmetric dihydroxylation of stilbene was carried out as described in Example 1, except that 1.2 equivalents of NMO, as a 62% wt. solution in water, were used.

EXAMPLE 4 Preparation of dihydroquinidine derivative

Preparation of dihydroquinidine by catalytic reduction of quinidine

To a solution of 16.2 g of quinidine (0.05mol) in 150mL of 10% $H_2SO_4$ (15 g conc $H_2SO_4$ in 150mL $H_2O$) was added 0.2 g of $PdCl_2$ (0.022eq; 0.0011mol). The reaction mixture was hydrogenated in a Parr shaker at 50 psi pressure. After 2h, the catalyst was removed by filtration through a pad of celite and washed with 150mL of water. The faint yellow solution so obtained was slowly added to a stirred aqueous NaOH solution (15 g of NaOH in 150mL $H_2O$. A white precipitate immediately formed and the pH of the

solution was brought to 10-11 by addition of excess aqueous 15% NaOH. The precipitate was collected by filtration, pressed dry and suspended in ethanol (175mL). The boiling solution was quickly filtered and upon cooling to room temperature, white needles crystallized out. The crystals were collected and dried under vacuum (90°C; 0.05 mm Hg) overnight. This gave 8.6 g (52.7%) of pure dihydroquinidine mp = 169.5-170°C. The mother liquor was placed in a freezer at -15°C overnight. After filtration and drying of the crystals, another 4.2 g (21.4%) of pure material was obtained, raising the total amount of dihydroquinidine to 12.8 g (74.1%).

Preparation of dihydroquinidine p-chlorobenzoate (ligand 1)

From dihydroquinidine hydrochloride (Aldrich)

To a cooled (0°C) suspension of 100 g dihydroquinidine hydrochloride (0.275mol) in 300mL of dry $CH_2Cl_2$ was added, over 30 minutes with efficient stirring, 115mL of $Et_3N$ (0.826eq; 3eqs) dissolved in 50mL of $CH_2Cl_2$. The dropping funnel was rinsed with an additional 20mL of $CH_2Cl_2$. After stirring 30 minutes at 0°C, 42mL of p-chlorobenzoyl chloride (0.33mol;57.8g; 1.2eq) dissolved in 120mL of $CH_2Cl_2$ was added dropwise over a period of 2h. The heterogeneous reaction mixture was then stirred 30 minutes at 0°C and 1 hour at room temperature; 700mL of a 3.0M NaOH solution was then slowly added until pH = 10-11 was obtained. After partitioning, the aqueous layer was extracted with three 100mL portions of $CH_2Cl_2$. The combined organic layers were dried over $Na_2SO_4$ and the solvent removed in vacuo (rotatory evaporator). The crude oil was dissolved in 1L of ether, cooled to 0°C and treated with HCl gas until the ether solution gives a pH of about 2 using wet pH paper. The slightly yellow precipitate was collected and dried under vacuum to give 126 g (91.5%) of dihydroquinidine p-chlorobenzoate hydrochloride.

The salt was suspended in 500mL of ethyl acetate, cooled to 0°C and treated with 28% $NH_4OH$ until pH = 11 was reached. After separation, the aqueous layer was extracted with two 200mL portions of ethyl acetate. The combined organic layers were dried over $Na_2SO_4$ and the solvent removed under vacuum, leaving the free base 1 as a white foam (112g; 88% overall). This material can be used without further purification, or it can be recrystallized from a minimum volume of hot acetonitrile to give an approximately 70-80% recovery of colorless crystals: mp: 102-104°C, $[\alpha]^{25}D$-76.5°[c1.11, EtOH); IR ($CH_2Cl_2$) 2940, 2860, 1720, 1620, 1595, 1520, 1115, 1105, 1095, 1020 $cm^{-1}$; $^1H$ NMR ($CDCl_3$) 8.72 (d, 1H, J = 5Hz), 8.05 (br d, 3H, J = 9.7Hz), 7.4 (m, 5H), 6.72 (d, 1H, J = 7.2Hz), 3.97 (s, 3H), 3.42 (dd, 1H, J = 9, 19.5Hz), 2.9-2.7 (m, 4H), 1.87 (m, 1H), 1.75 (br s, 1H), 1.6-1.45 (m, 6H), 0.92 (t, 3H, J = 7Hz). Anal. Calcd for $C_{27}H_{29}ClN_2O_3$: C, 69.74; H, 6.28; Cl, 7.62; N, 6.02. Found: C, 69.95;H, 6.23; Cl, 7.81; N, 5.95.

From dihydroquinidine

To a 0°C solution of 1.22g dihydroquinidine (0.0037mol) in 30mL of $CH_2Cl_2$ was added 0.78mL of $Et_3N$ (0.0056mol; 1.5eq), followed by 0.71mL of p-chlorobenzoyl chloride (0.005mol; 1.2eq) in 1mL $CH_2Cl_2$. After stirring 30 minutes at 0°C and 1 hour at room temperature, the reaction was quenched by the addition of 10% $Na_2CO_3$ (20mL). After separation, the aqueous layer was extracted with three 10mL portions of $CH_2Cl_2$. The combined organic layers were dried over $Na_2SO_4$ and the solvent removed under vacuum. The crude product was purified as described above. Dihydroquinidine p-chlorobenzoate (1) was obtained in 91% yield (1.5g) as a white foam.

Recovery of dihydroquinidine p-chlorobenzoate

The aqueous acidic extracts (see EXAMPLE 1) were combined, cooled to 0°C and treated with 2.0M NaOH solution (500mL) until pH = 7 was obtained. Methylene chloride was added (500mL) and the pH was adjusted to 10-11 using more 2.0M NaOH. The aqueous layer was separated and extracted with two 300mL portions of $CH_2Cl_2$. The combined organic layers were dried over $Na_2SO_4$ and concentrated to leave the crude alkaloid as a yellow foam. The crude dihydroquinidine p-chlorobenzoate (1) was dissolved in 1L of ether, cooled to 0°C and HCl gas was bubbled into the solution until a pH of 1-2 was obtained using wet pH paper. The pale yellow precipitate of 1 as the hydrochloride salt was collected by filtration and dried under high vacuum (0.01mm Hg). The free base was liberated by suspending the salt in 500mL of ethyl acetate, cooling the heterogeneous mixture to 0°C and adding 28% $NH_4OH$ (or 15% NaOH) until pH = 11 was obtained. After separation, the aqueous layer was extracted with two 100mL portions of ethyl acetate, the combined organic layers were dried over $Na_2SO_4$ and the solvent removed in vacuo to give 56g (91% recovery) of pure dihydroquinidine p-chlorobenzoate (1) as a white foam.

EXAMPLE 5 Preparation of dihydroquinine derivative

Preparation of dihydroquinine p-chlorobenzoate

The catalytic hydrogenation and p-chlorobenzoylation were conducted as described for the dihydroquinidine p-chlorobenzoate to give a white amorphous solid in 85-90% yield. This solid can be used without further purification, or it can be recrystallized from a minimum volume of hot acetonitrile to afford colorless crystals: Mp:130-133°C, $[\alpha]^{25}D+150°$ (c 1.0, EtOH). The physical properties of the solid before recrystallization (i.e., the "white amorphous solid") are as follows: $[\alpha]^{25}$ D+142.1 (C=1, EtOH); IR ($CH_2Cl_2$) 2940, 2860, 1720, 1620, 1595, 1508, 1115, 1105, 1095, 1020 $cm^{-1}$, $^1H$ NMR ($CDCl_3$) d 8.72 (d, 1H, J=5Hz), 8.05 (br d, 3H, J=8Hz), 7.4 (m, 5H), 6.7 (d, 1H, J=8Hz), 4.0 (s, 3H), 3.48 (dd, 1H, J=8.5, 15.8Hz), 3.19 (m, 1H), 3.08 (dd, 1H, J=11, 15Hz), 2.69 (ddd, 1H, J=5, 12, 15.8Hz), 2.4 (dt, 1H, J=2.4, 15.8Hz), 1.85-1.3 (m, 8H), 0.87 (t, 3H, J=Hz). Anal. Calcd for $C_{27}H_{29}ClN_2O_3$ : C, 69.74; H, 6.28; Cl, 7.62; N, 6.02. Found: C, 69.85; H, 6.42; Cl, 7.82; N, 5.98.

Recovery of dihydroquinine p-chlorobenzoate (2)

The procedure is identical to that described above for recovery of 1.

EXAMPLE 6 Procedure for Asymmetric Dihydroxylation of Trans-3-hexene Under "Slow Addition" Conditions

To a well stirred mixture of 0.465g (1 mmol, 0.25 eq=0.25M in L) dihydroquinidine 4-chlorobenzoate (Aldrich, 98%), 0.7g (6 mmol, 1.5 eq) N-methylmorpholine N-oxide (Aldrich, 97%), and 32 L of a 0.5M toluene solution of osmium tetroxide (16 mol, 4 x $10^{-3}$ equiv), in 4 mL of an acetone-water mixture (10:1 v/v) at 0°C, neat 0.5 mL (0.34g, 4 mmol) trans-3-hexene (Wiley, 99.9%) was added slowly, via a gas tight syringe controlled by a syringe pump and with the tip of the syringe needle immersed in the reaction mixture, over a period of 16 h. The mixture gradually changed from heterogeneous to homogeneous. After the addition was complete, the resulting clear orange solution was stirred at 0°C for an additional hour. Solid sodium metabisulfite ($Na_2S_2O_5$, 1.2g) was added and the mixture was stirred for 5 min, and then diluted with dichloromethane (8mL) and dried ($Na_2SO_4$). The solids were removed by filtration, and washed three times with dichloromethane. The combined filtrates were concentrated, and the residual oil was subjected to flash column chromatography on silica gel (25g, elution with diethyl ether-dichloromethane, 2:3 v/v, $R_f$ 0.33) and collection of the appropriate fractions afforded 0.30-0.32g (85-92% yield) of the hexanediol. The enantiomeric excess of the diol was determined by GLC analysis (5% phenyl-methylsilicone, 0.25 m film, 0317 mm diameter, 29 m long) of the derived bis-Mosher ester to be 70%.

When the above reaction was repeated with 1.2 mL (6mmol, 1.5eq) 60% aqueous NMO (Aldrich) in 4 mL acetone, an ee of 71% was obtained. Thus, this aqueous NMO gives equivalent results and is almost twenty times less expensive than the 97% solid grade. With an alkaloid concentration of only 0.1M (i.e., 0.186g) and with an olefin addition period of 20 hours at 0°C, the ee was 65%. A small sacrifice in ee thus leads to a large saving in alkaloid. At 0°C, both trans-3-hexene and trans-$\beta$-methylstyrene reach their maximum ee value between 0.20 and 0.25M alkaloid concentration.

EXAMPLE 7 Asymmetric Dihydroxylation of 1-Phenylcyclohexene with $Et_4NOAc$-$4H_2O$

The procedure set out in EXAMPLE 1 was followed, except that 1-phenylcyclohexene (1.0M) was substituted for trans-stilbene. The reaction was allowed to proceed for three days, after which only 40% conversion to the diol was obtained (8% ee).

The above procedure was repeated, with the difference that 2 equivalents of tetraethyl ammonium acetate ($Et_4NOAc$-$4H_2O$) was added to the reaction mixture at the beginning of the reaction. Fifty-two (52%) percent ee was obtained using this procedure, and the reaction was finished in about one day.

EXAMPLE 8 Asymmetric Dihydroxylation of trans-Stilbene under "phase-transfer" conditions in toluene

To a well-stirred mixture of 58.2 mg (0.125 mmol; 0.25 eq.) of the p-chlorobenzoate of hydroquinidine; 1 mL of toluene, 88 mg (0.75 mmol; 1.5 eq.) of N-methylmorpholine N-oxide, 181 mg (1 mmol; 2 eq.) of tetramethylammonium hydroxide pentahydrate, 57 $\mu$L (2 mmol; 2 eq.) of acetic acid, 0.1 mL of water, and $OsO_4$ (4.2 $\mu$L of solution prepared using 121 mg $OsO_4$/mL toluene; 0.004 Mol%, 0.004 eq.) at room

temperature, a toluene solution (1 mL) of 90 mg (0.4 mmol) of trans-stilbene was added slowly, with a gas-tight syringe controlled by a syringe pump and with the tip of the syringe needle immersed in the reaction mixture, over a period of 24 h. After the addition was completed, 10% $NaHSO_3$ solution (2.5 mL) was added to the mixture, and the resulting mixture was stirred for 1 h. Organic materials were extracted with ethyl acetate, and the combined extracts were washed with brine and dried over $Na_2SO_4$. The solvent was evaporated under reduced pressure, and the residual oil was subjected to column chromatography on silica gel (5 g, elution with hexane-ethyl acetate, 2:1 $^V/_V$, $R_f$0.17) to afford 67.3 mg (63%) of the diol. The enantiomeric excess of the diol was determined by HPLC analysis of the derived bis-acetate (Pirkle 1A column using 5% isopropanol/hexane mixture as eluant. Retention times are: $t_1$ = 22.6 minutes; $t_2$ = 23.4 minutes) to be 94%.

EXAMPLE 9 Asymmetric Dihydroxylation of trans-Methyl 4-methoxycinnamate under phase-transfer conditions in toluene

To a well-stirred mixture of 116.3 mg (0.25 eq.) of the p-chlorobenzoate of hydroquinidine, 2 mL of toluene, 175.8 mg (1.5 mmol; 1.5 eq.) of N-methylmorpholine N-oxide, 522 mg (2 mmol; 2 eq.) of tetraethylammonium acetate tetrahydrate, 0.2 mL of water, and $OsO_4$ (8.4 µL of solution prepared using 121 mg $OsO_4$/mL toluene; 0.004 Mol%, 0.004 eq.) at room temperature, a toluene solution (1 mL) of 192 mg (1 mmol) of trans-methyl 4-methoxycinnamate was added slowly, with a gas-tight syringe controlled by a syringe pump and with the tip of the syringe needle immersed in the reaction mixture, over a period of 24 h. After the addition was complete, 10% $NaHSO_3$ solution (5 mL) was added to the mixture, and the resulting mixture was stirred for 1 h. Organic materials were extracted with ethyl acetate, and the combined extracts were washed with brine and dried over $Na_2SO_4$. The solvent was evaporated under deduced pressure, and the residual oil was subjected to column chromatography on silica gel (10 g, elution with hexane-ethyl acetate, 2:1$^V$/V, $R_f$ 0.09) to afford 118.8 mg (53%) of the diol. The enantiomeric excess of the diol was determined by HPLC analysis of the derived bis-acetate (Pirkle Covalent Phenyl Glycine column using 10% isopropanol/hexane mixture as eluant. Retention times are: $t_1$ = 25.9 minutes; $t_2$ = 26.7 minutes) to be 84%.

EXAMPLE 10 Asymmetric Dihydroxylation of trans-Stilbene in the presence of Boric Acid

To a well-stirred mixture of 58.2 mg (0.125 mmol; 0.25 eq) of the p-chlorobenzoate of hydroquinidine, 70 mg (0.6 mmol; 1.2 eq.) of N-methylmorpholine N-oxide, 37 mg (0.6 mmol; 1.2 eq.) of boric acid, 0.5 mL of dichloromethane, and $OsO_4$ (4.2 µL of a solution prepared using 121 mg $OsO_4$/mL toluene; 0.004 Mol%, 0.004 eq.) at room temperature, a dichloromethane solution (1 mL) of 90 mg (0.5 mmol) of trans-stilbene was added slowly, with a gas-tight syringe controlled by a syringe pump and with the tip of the syringe needle immersed in the reaction mixture, over a period of 24 h. After the addition was complete, 10% $NaHSO_3$ solution (2.5 mL) was added to the mixture, and the resulting mixture was stirred for 1 h. Organic materials were extracted with ethyl acetate, and the combined extracts were washed with brine and dried over $Na_2SO_4$. The solvent was evaporated under reduced pressure, and the residual oil was subjected to column chromatography on silica gel (5 g, elution with hexane-ethyl acetate, 2:1 $^V/_V$, $R_f$ 0.17) to afford 78.3 mg (73%) of the diol. The enantiomeric excess of the diol was determined by $^1$H-NMR (solvent: $CDCl_3$) analysis of the derived bis-Mosher ester to be 94%.

EXAMPLE 11 Asymmetric Dihydroxylation of trans-Methyl 4-methoxycinnamate in the presence of Boric Acid

To a well-stirred mixture of 116.3 mg (0.25 mmol; 0.25 eq.) of the p-chlorobenzoate of hydroquinidine, 175.8 mg (1.5 mmol; 1.5 eq.) of N-methylmorpholine N-oxide, 74.4 mg (1.2 mmol; 1.2 eq.) of boric acid, 1 mL of dichloromethane, and $OsO_4$ (8.4 µL of a solution prepared using 121 mg $OsO_4$/mL toluene, 0.004 mol%, 0.004 eq.) at room temperature, a dichloromethane solution (1mL) of 192 mg (1 mmol) of trans-methyl 4-methoxycinnamate was added slowly, with a gas-tight syringe controlled by a syringe pump and with the tip of the syringe needle immersed in the reaction mixture, over a period of 24 h. After the addition was complete, 10% $NaHSO_3$ solution (5 mL) was added to the mixture, and the resulting mixture was stirred for 1 h. Organic materials were extracted with ethyl acetate, and the combined extracts were washed with brine and dried over $Na_2SO_4$. The solvent was evaporated under reduced pressure, and the residual oil was subjected to column chromatography on silica gel (10 g, elution with hexane-ethyl acetate, 2:1 $^V/_V$, $R_f$ 0.09) to afford 151.1 mg (67%) of the diol. The enantiomeric excess of the diol was determined by HPLC

analysis of the derived bis-acetate (Pirkle Covalent Phenyl Glycine column using 10% isopropanol/hexane mixture as eluant. Retention times are: $t_1$ = 24.0 minutes; $t_2$ = 24.7 minutes) to be 76%.

EXAMPLE 12 Asymmetric Dihydroxylation of trans-β-Methylstyrene in the presence of Boric Acid

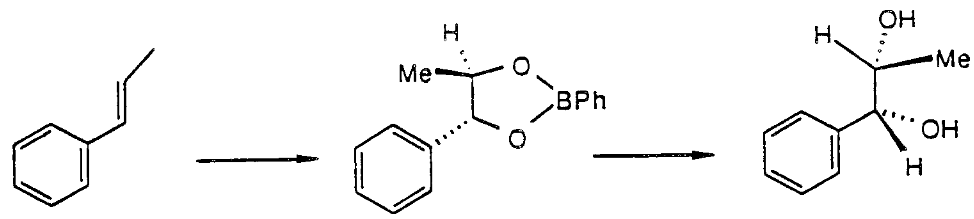

To a well-stirred mixture of 58.2 mg (0.125 mmol; 0.25 eq) of the p-chlorobenzoate of hydroquinidine, 70 mg (0.6 mmol; 1.2 eq) of N-methylmorpholine N-oxide, 72 mg (0.6 mmol; 1.2 eq) of phenylboric acid, 0.5 mL of dichloromethane, and $OsO_4$ (4.2 μL [of a solution prepared using 121 mg $OsO_4$/mL toluene; 0.004 Mol%, 0.004 eq) at 0 °C, a dichloromethane solution] (0.5 mL), 65μL (0.5 mmol) trans-β-methylstyrene was added slowly, with a gas-tight syringe controlled by a syringe pump and with the tip of the syringe needle immersed in the reaction mixture, over a period of 24 h. After the addition was complete, 10% $NaHSO_3$ solution (2.5 mL) was added to the mixture, and the resulting mixture was stirred for 1 h. Organic materials were extracted with ethyl acetate, and the combined extracts were washed with brine and dried over $Na_2SO_4$. The solvent was evaporated under reduced pressure, and the residual oil was subjected to column chromatography on silica gel (5 g, elution with hexane-ethyl acetate, 2:1 $^V/_V$, $R_f$ 0.62) to afford 109 mg (91%) of the phenylborate. The phenylborate was dissolved into acetone (3 mL) and 1,3-propandiol (0.5 mL), and the resulting mixture was stood for 2 h at room temperature. The solvent was evaported under reduced pressure, and the residual oil was subjected to column chromatography on silica gel (5g, elution with hexaneethyl acetate, 2:1 $^V/_V$, $R_f$ 0.10) to afford 48.6 mg (70%) of the diol. The enantiomeric excess of the diol was determined by HPLC analysis of the derived bis-acetate (Pirkle 1A column using 0.5% isopropanol/hexane mixture as eluant. Retention times are: $t_1$ = 17.1 minutes; $t_2$ = 18.1 minutes) to be 73%.

Equivalents

Those skilled in the art will recognize, or be able to ascertain, using no more than routine experimentation, numerous equivalents to the specific substances and procedures described herein. Such equivalents are considered to be within the scope of this invention, and are covered by the following claims.

## Claims

**1.** A method of osmium-catalyzed asymmetric addition to an olefin, comprising the step of combining the olefin, a chiral ligand (for example a cinchona alkaloid, e.g. dihydroquinine or dihydroquinidine, or derivatives thereof), an oxidant and an osmium-containing catalyst in which either (a) the olefin (which may be dissolved in an organic solvent) is slowly added to the combination and/or (b) a soluble carboxylate salt, for example a tetraalkyl (e.g. tetraethyl) ammonium carboxylate (e.g. acetate) is added.

**2.** A method according to claim 1, wherein the asymmetric addition is asymmetric dihydroxylation, the chiral ligand is a cinchona alkaloid or a derivative thereof, the organic solvent is e.g. acetone, dichloromethane or chloroform, the oxidant is an amine oxide (e.g. N-methylmorpholine N-oxide) and the osmium containing catalyst is e.g. osmium tetroxide.

**3.** A method according to claim 2, wherein the combining step comprises the steps of:
a. combining the cinchona alkaloid or derivative thereof, the organic solvent, water and the amine oxide, the alkaloid being present eg. in a concentration from 0.01M to 2.0M;
b. adding the osmium-containing catalyst to the combination formed in (a);
c. adding the olefin slowly to the combination formed in (b); and

d. maintaining the resulting combination produced in (c) under conditions appropriate for asymmetric dihydroxylation of the olefin to occur.

**4.** A method according to claims 2 or 3, further comprising adding boric acid or a derivative thereof, eg. in step (a) of claim 3.

**5.** A method according to claim 1, wherein the asymmetric addition is asymmetric oxyamination, the chiral ligand is a cinchona alkaloid or a derivative thereof, and the oxidant is a metallochloramine derivative.

**6.** A method according to claim 1, wherein the asymmetric addition is asymmetric diamination, the chiral ligand is a cinchona alkaloid or a derivative thereof, and the oxidant is a metallochloramine derivative, and wherein the olefin is added slowly to a combination of the alkaloid, organic solvent, water, oxidant, catalyst and an amine, under conditions appropriate to asymmetric diamination.

**7.** A method according to claim 1, wherein the chiral ligand and osmium-containing catalyst are in the form of an osmium-alkaloid catalyst complex, the complex comprising an osmium-containing composition and an alkaloid or derivative thereof.

**8.** A method according to claim 1, wherein the organic solvent is acetone, the oxidant is selected from amine oxides, hydrogen peroxide, tert-butyl hydroperoxide, a metal catalyst/oxygen combination N-chloro-N-metallo carbamates and chloramine-T, and the osmium-containing compound is osmium tetroxide.

**9.** A chiral ligand selected from:
a. dimethylcarbamoyl dihydroquinidine;
b. benzoyl dihydroquinidine;
c. 4-methoxybenzoyl dihydroquinidine;
d. 4-chlorobenzoyl dihydroquinidine;
e. 2-chlorobenzoyl dihydroquinidine;
f. 4-nitrobenzoyl dihydroquinidine;
g. 3-chlorobenzoyl dihydroquinidine;
h. 2-methoxybenzoyl dihydroquinidine;
i. 3-methoxybenzoyl dihydroquinidine;
j. 2-naphtoyl dihydroquinidine;
k. cyclohexanoyl dihydroquinidine;
l. p-phenylbenzoyl dihydroquinidine;
m. dimethylcarbamoyl dihydroquinine;
n. benzoyl dihydroquinine;
o. 4-methoxybenzoyl dihydroquinine;
p. 4-chlorobenzoyl dihydroquinine;
q. 2-chlorobenzoyl dihydroquinine;
r. 4-nitrobenzoyl dihydroquinine;
s. 3-chlorobenzoyl dihydroquinine;
t. 2-methoxybenzoyl dihydroquinine;
u. 3-methoxybenzoyl dihydroquinine;
v. 2-naphtoyl dihydroquinine;
w. cyclohexanoyl dihydroquinine;
x. p-phenylbenzoyl dihydroquinine;
y. methoxydihydroquinidine; and

a dihydroquinidine ester of the formula

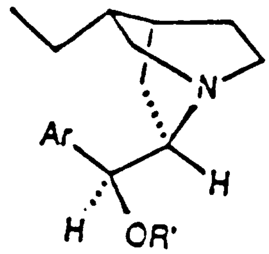

wherein R' is p-chlorobenzoyl and Ar is

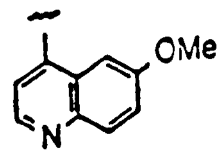

a dihydroquinine ester of the formula

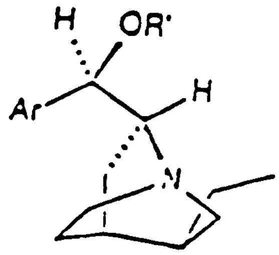

wherein R' is p-chlorobenzoyl and Ar is

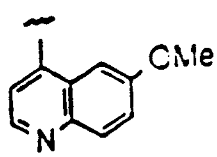

**10.** A method according to any one of claims 1 to 8, wherein the chiral ligand is selected from the chiral ligands of claim 9.

**Patentansprüche**

**1.** Verfahren zur osmium-katalysierten asymmetrischen Addition an Olefine, das den Schritt des Zusammengebens des Olifins, eines chiralen Liganten (zum Beispiel eines Cinchona-Alkaloids, wie eines Dihydrochinins oder Dihydrochinidins oder eines Derivates davon), eines Oxidationsmittels und eines Osmium enthaltenen Katalysator umfasst, in dem entweder (a) das Olefin (welches in einem organischen Lösungsmittel gelöst sein kann) langsam zu der Kombination und/oder (b) ein lösliches Karbonsäuresalz, z.B. ein Tetraalkyl-(zB. Tetraethyl)-ammonium-carboxylat (z.B. Acetat) hinzugefügt wird.

2. Ein Verfahren gemäß Anspruch 1, worin die asymmetrische Addition eine asymmetrische Dihydroxylation ist, der chirale Ligant ein Cinchona-Alkaloid oder ein Derivat davon ist, das organische Lösungsmittel z.B. Aceton, Dichlormethan oder Chloroform ist, das Oxidationsmittel ein Aminoxid (z.B. N-Methylmorpholin-N-oxid) ist und der Osmium enthaltene Katalysator z.B. Osmiumtetroxide ist.

3. Ein Verfahren gemäß Anspruch 2 worin der Schritt des Zusammengebens folgende Schritte umfaßt:
   a. Zusammengeben des Chinchona-Alkaloids oder eines Derivat davon, des organischen Lösungsmittels, Wasser, und des Aminoxids, wobei das Alkaloid in einer Konzentration von 0,1M - 2.0M anwesend ist;
   b. Hinzufügen des Osmium enthaltenen Katalysators zu der Kombination gebildet in (a);
   c. Langsames Hinzufügen des Olefins zu der Kombination gebildet in (b); und
   d. Halten der in (c) gebildeten Kombination unter Bedingungen, die geeignet sind, daß damit die asymmetrische Dihydroxylation des Olefins vonstatten geht.

4. Ein Verfahren gemäß Anspruch 2 oder Anspruch 3, das weiterhin das Hinzufügen von Borsäure oder eines Derivates davon in Schritt (a) des Anspruchs 3 umfaßt.

5. Ein Verfahren gemäß Anspruch 1, worin die asymmetrische Addition eine asymmetrische Oxyamination, der chirale Ligant ein Cinchona-Alkaloid oder ein Derivat davon, und das Oxidationsmittel ein Metallchloramin-Derivat ist.

6. Ein Verfahren gemäß Anspruch 1, worin die asymmetrische Addition eine asymmetrische Diamination ist, der chirale Ligant ein Cinchona-Alkaloid oder ein Derivat davon ist und das Oxidationsmittel ein Metallchloramin-Derivat ist, und wobei das Olefin langsam zu einer Kombination des Alkaloids, des organischen Lösungsmittels, Wasser, des Oxidationsmittels, des Katalysators und eines Amins gegeben wird unter Bedingungen, die für die asymmetrische Diamination geeignet sind.

7. Ein Verfahren gemäß Anspruch 1, worin der chirale Ligant und der Osmium enthaltene Katalysator in der Form eines Osmium-Alkaloid-Katalysatorkomplexes vorliegen, wobei der Komplex eine Osmium enthaltende Kombination und ein Alkaloid oder Derivat davon umfaßt.

8. Ein Verfahren gemäß Anspruch 1, worin das organische Lösungsmittel Aceton ist, das Oxidationsmittel ausgewählt ist aus Aminoxiden, Wasserstoffperoxid, tert-Butyl-hydroperoxid, einer Metallkatalysator/Sauerstoff-Kombination, N-Chlor-N-metall-carbamate und Chloramin-T, und die Osmium enthaltende Verbindung Osmiumtetroxid ist.

9. Ein chiraler Ligant ausgewählt aus:
   a. Dimethylcarbamoyl-dihydrochinidin;
   b. Benzoyl-dihydrochinidin;
   c. 4-Methoxybenzoyl-dihydrochinidin;
   d. 4-Chlorbenzoyl-dihydrochinidin;
   e. 2-Chlorbenzoyl-dihydrochinidin;
   f. 4-Nitrobenzoyl-dihydrochinidin;
   g. 3-Chlorbenzoyl-dihydrochinidin;
   h. 2-Methoxybenzoyl-dihydrochinidin;
   i. 3-Methoxybenzoyl-dihydrochinidin;
   j. 2-Naphtoyl-dihydrochinidin;
   k. Cyclohexanoyl-dihydrochinidin;
   l. p-Phenylbenzoyl-dihydrochinidin;
   m. Dimethylcarbamoyl-dihydrochinin;
   n. Benzoyl-dihydrochinin;
   o. 4-Methoxybenzoyl-dihydrochinin;
   p. 4-Chlorbenzoyl-dihydrochinin;
   q. 2-Chlorbenzoyl-dihydrochinin;
   r. 4-Nitrobenzoyl-dihydrochinin;
   s. 3-Chlorbenzoyl-dihydrochinin;
   t. 2-Methoxybenzoyl-dihydrochinin;
   u. 3-Methoxybenzoyl-dihydrochinin;

v. 2-Naphtoyl-dihydrochinin;
w. Cyclohexanoyl-dihydrochinin;
x. p-Phenylbenzoyl-dihydrochinin;
y. Methoxydihydrochinidin und

Ein Dihydrochinidinester der Formel

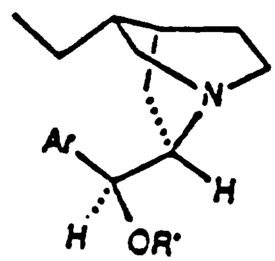

worin R' p-Chlorbenzoyl ist und Ar

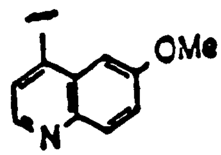

ist, oder ein Dihydrochininester der Formel

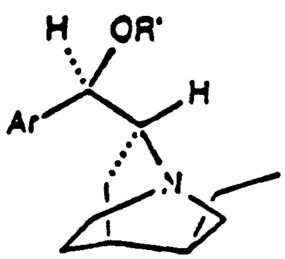

worin R' p-Chlorbenzoyl und Ar

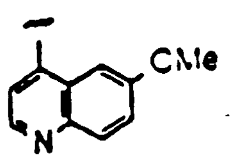

ist.

**10.** Ein Verfahren gemäß einem der Ansprüche 1 bis 8. worin der chirale Ligant ausgewählt ist aus den chiralen Liganten des Anspruchs 9.

**Revendications**

**1.** Procédé d'addition asymétrique, catalysée par l'osmium, à une oléfine, comprenant l'étape de combinaison de l'oléfine, d'un ligand chiral (par exemple un alcaloïde de quinquina, comme la dihydroquinine ou la dihydroquinidine, ou leurs dérivés), un oxydant et un catalyseur contenant de l'osmium où soit (a)

l'oléfine (qui peut être dissoute dans un solvant organique) est ajoutée lentement à la combinaison et/soit (b) un sel carboxylate soluble, par exemple un carboxylate (comme acétate) de tétraalkyl-(par exemple tétraéthyl) ammonium est ajouté.

**2.** Procédé selon la revendication 1, où l'addition asymétrique est une dihydroxylation asymétrique, le ligand chiral est un alcaloïde de quinquina ou un dérivé de celui-ci, le solvant organique est par exemple l'acétone, le dichlorométhane ou le chloroforme, l'oxydant est un oxyde d'amine (par exemple le N-oxyde de N-méthylmorpholine) et le catalyseur contenant de l'osmium est par exemple le tétroxyde d'osmium.

**3.** Procédé selon la revendication 2, où l'étape de combinaison comprend les étapes de :
- a. combinaison de l'alcaloïde de quinquina ou de son dérivé, du solvant organique, de l'eau et de l'oxyde d'amine, l'alcaloïde étant présent par exemple à une concentration de 0,01 M à 2,0 M ;
- b. addition du catalyseur contenant de l'osmium à la combinaison formée en (a) ;
- c. addition lentement de l'oléfine à la combinaison formée en (b) ; et
- d. maintien de la combinaison obtenue produite en (c) dans des conditions appropriées pour qu'une dihydroxylation asymétrique de l'oléfine se produise.

**4.** Procédé selon les revendications 2 ou 3, comprenant de plus l'addition d'acide borique ou d'un dérivé de celui-ci dans l'étape (a) de la revendication 3.

**5.** Procédé selon la revendication 1, où l'addition asymétrique est une oxyamination asymétrique, le ligand chiral est un alcaloïde de quinquina ou un dérivé de celui-ci et l'oxydant est un dérivé de métallochloramine.

**6.** Procédé selon la revendication 1, où l'addition asymétrique est une diamination asymétrique, le ligand chiral est un alcaloïde de quinquina ou un dérivé de celui-ci et l'oxydant est un dérivé de métallochloramine, et où l'oléfine est ajoutée lentement à une combinaison de l'alcaloïde, du solvant organique, de l'eau, de l'oxydant, du catalyseur et d'une amine, dans des conditions appropriées à une diamination asymétrique.

**7.** Procédé selon la revendication 1, où le ligand chiral et le catalyseur contenant de l'osmium sont sous forme d'un complexe catalytique osmium-alcaloïde, le complexe comprenant une composition contenant de l'osmium et un alcaloïde ou un dérivé de celui-ci.

**8.** Procédé selon la revendication 1, où le solvant organique est l'acétone, l'oxydant est choisi parmi les oxydes d'amine, le peroxyde d'hydrogène, l'hydroperoxyde de tert-butyle, une combinaison catalyseur métallique/oxygène, les N-chloro-N-métallocarbamates et la chloramine-T, et le composé contenant de l'osmium est le tétroxyde d'osmium.

**9.** Ligand chiral choisi parmi :
- a. la diméthylcarbamoyldihydroquinidine ;
- b. la benzoyldihydroquinidine ;
- c. la 4-méthoxybenzoyldihydroquinidine ;
- d. la 4-chlorobenzoyldihydroquinidine ;
- e. la 2-chlorobenzoyldihydroquinidine ;
- f. la 4-nitrobenzoyldihydroquinidine ;
- g. la 3-chlorobenzoyldihydroquinidine ;
- h. la 2-méthoxybenzoyldihydroquinidine ;
- i. la 3-méthoxybenzoyldihydroquinidine ;
- j. la 2-naphtoyldihydroquinidine ;
- k. la cyclohexanoyldihydroquinidine ;
- l. la p-phénylbenzoyldihydroquinidine ;
- m. la diméthylcarbamoyldihydroquinine ;
- n. la benzoyldihydroquinine ;
- o. la 4-méthoxybenzoyldihydroquinine ;
- p. la 4-chlorobenzoyldihydroquinine ;
- q. la 2-chlorobenzoyldihydroquinine ;

r. la 4-nitrobenzoyldihydroquinine ;
s. la 3-chlorobenzoyldihydroquinine ;
t. la 2-méthoxybenzoyldihydroquinine ;
u. la 3-méthoxybenzoyldihydroquinine ;
v. la 2-naphtoyldihydroquinine ;
w. la cyclohexanoyldihydroquinine ;
x. la p-phénylbenzoyldihydroquinine ;
y. la méthoxydihydroquinidine ; et

un ester de dihydroquinidine de formule

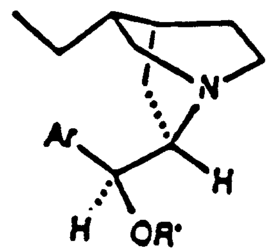

dans laquelle R' est un groupe p-chlorobenzoyle et Ar est

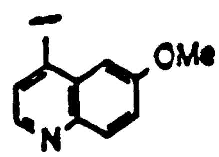

ou un ester de dihydroquinine de formule

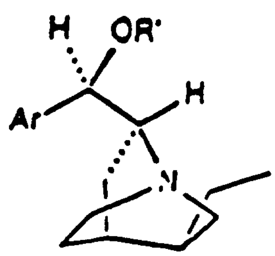

dans laquelle R' est un groupe p-chlorobenzoyle et Ar est

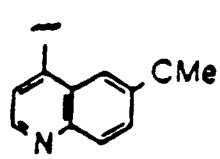

**10.** Procédé selon l'une quelconque des revendications 1 à 8, où le ligand chiral est choisi parmi les ligands chiraux de la revendication 9.

FIGURE 4

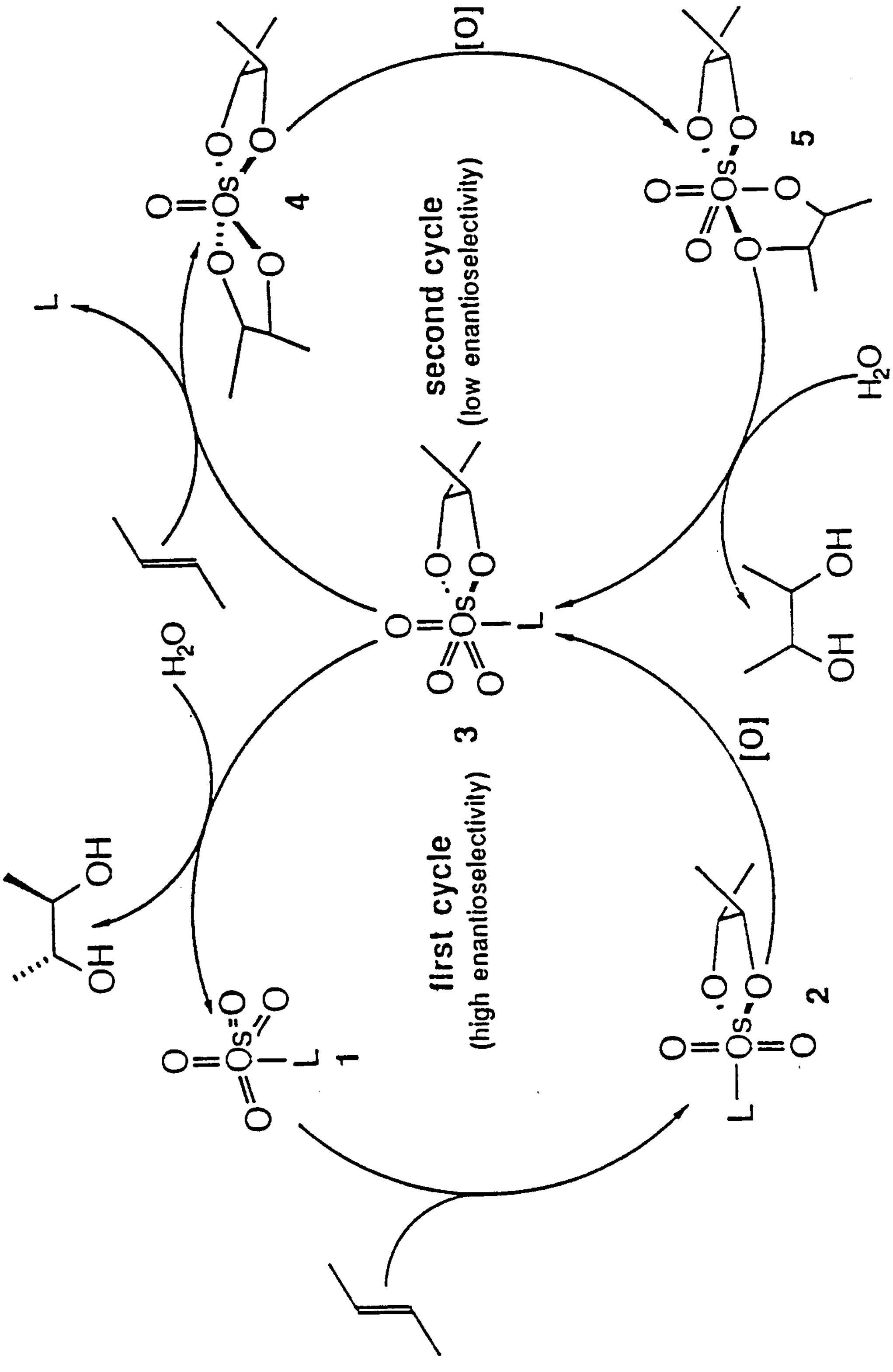


1 2 3 4 5

FIGURE 1

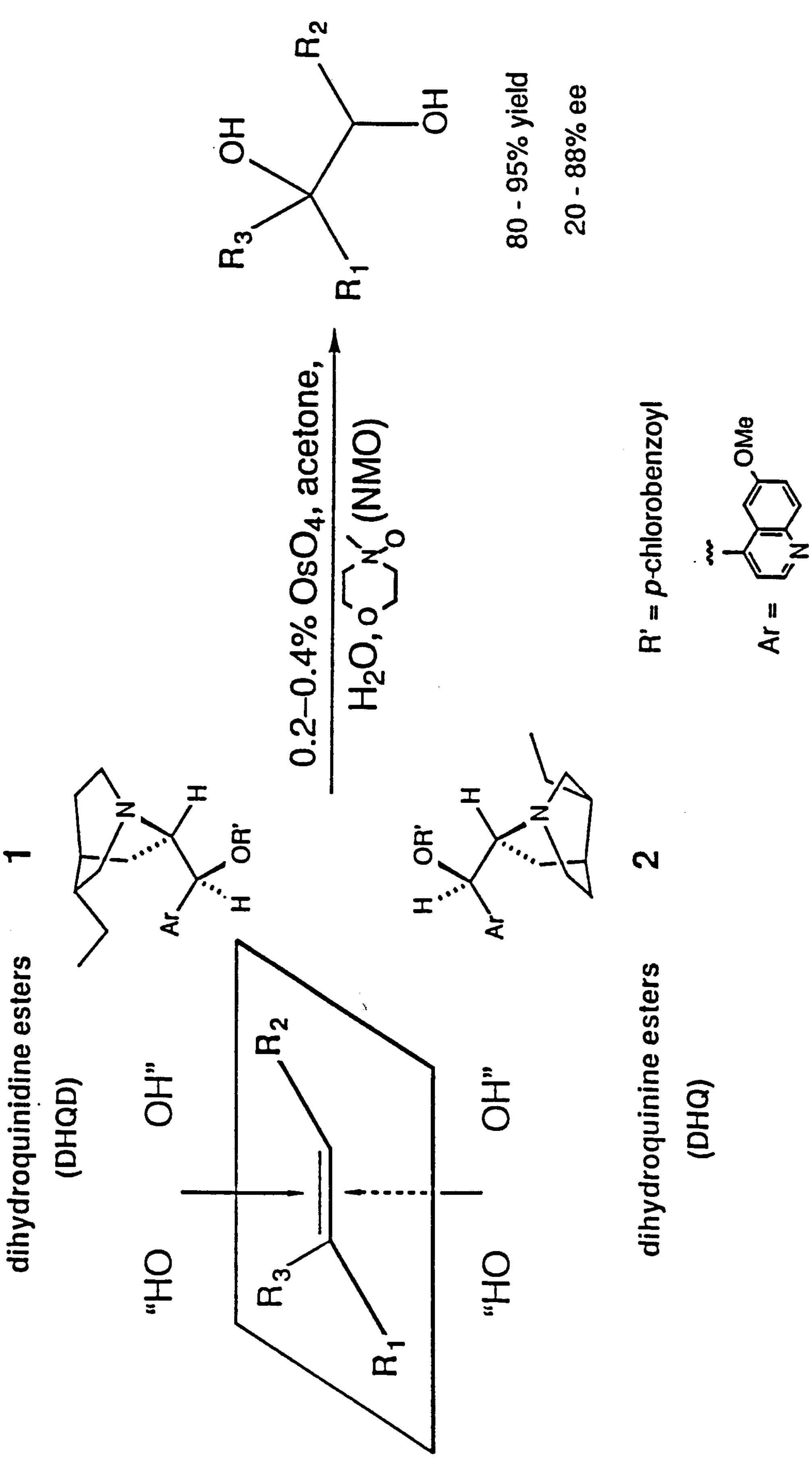


dihydroquinidine esters (DHQD) 1

dihydroquinine esters (DHQ) 2

R' = *p*-chlorobenzoyl

Ar =

FIGURE 2

## Asymmetric Catalytic Oxyamination

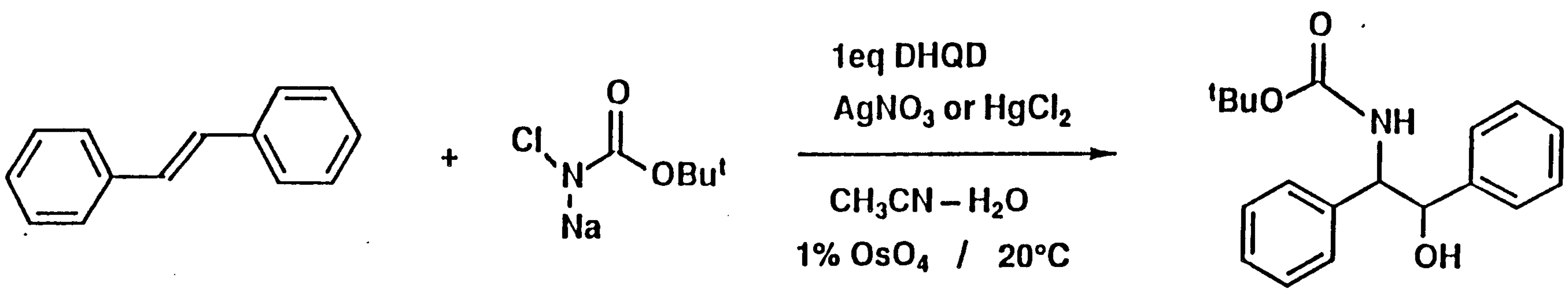


(51% ee)

DHQD =

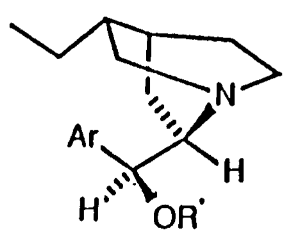

R' = *p*-chlorobenzoate

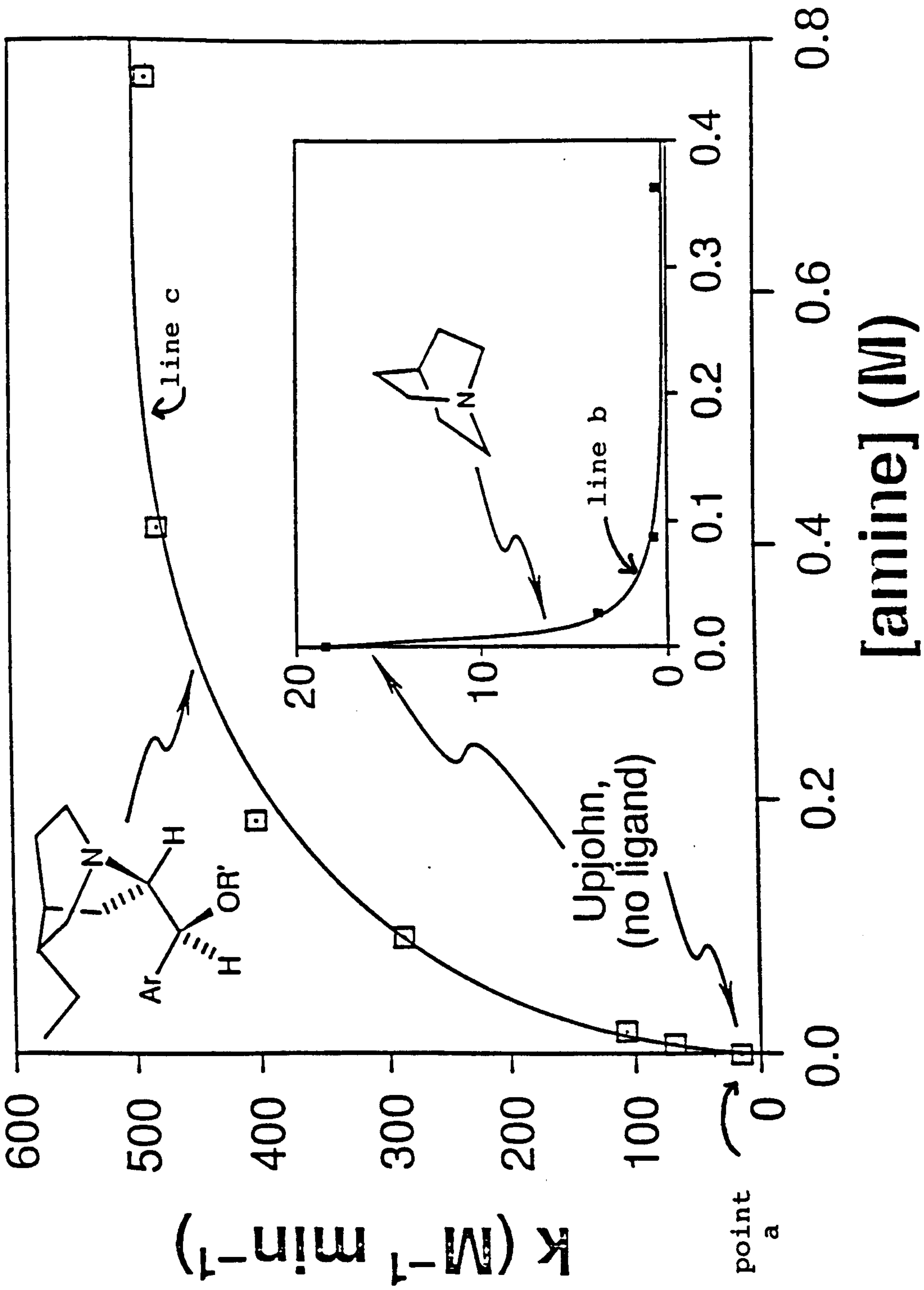
k (M⁻¹ min⁻¹)
[amine] (M)
0
100
200
300
400
500
600
0.0
0.2
0.4
0.6
0.8
0.0
0.1
0.2
0.3
0.4
20
10
line c
line b
Upjohn,
(no ligand)
point a

FIGURE 3